# EUROPÄISCHE PATENTSCHRIFT

(11) **EP 0 507 179 B1**
(45) Veröffentlichungstag und Bekanntmachung des Hinweises auf die Patenterteilung: **08.12.1999**
(21) Anmeldenummer: 92104979.7
(22) Anmeldetag: 23.03.1992
(51) Int. Cl.: C12N 15/86, C12N 15/38, A61K 39/245, C07K 14/00

(54) **Equine Herpesviren (EHV), die Fremd-DNA enthalten, Verfahren zu ihrer Herstellung und ihre Verwendung in Impfstoffen**
Equine herpes viruses (EHV) containing foreign DNA, process for their preparation and their use as vaccines
Virus de l'herpès équin (EHV) avec de l'ADN étranger, procédé pour leur préparation et leur utilisation comme vaccins

(30) Priorität: 05.04.1991 DE 4110962
(43) Veröffentlichungstag der Anmeldung: 07.10.1992
(73) Patentinhaber: BAYER AG, 51368 Leverkusen (DE)
(72) Erfinder: Darai, Gholamreza, Prof. Dr., W-6900 Heidelberg (DE); Thein, Peter, Prof. Dr. Dr. habil, W-8064 Oberzeitlbach (DE); Strube, Walter, Dr., W-5000 Köln 40 (DE); Ludwig, Hanns, Prof. Dr., W-1000 Berlin 37 (DE)

(56) Entgegenhaltungen:
- EP-A- 0 447 303
- WO-A-87/04463
- WO-A-92/01045
- WO-A-92/01057
- BIOTECHNOLOGY ABSTRACTS, DERWENT PUBLICATIONS LTD., LONDON, GB. ABSTRACT NO.91-05577 K.J. FAHEY 'Viruses as delivery vectors for vaccines - Recombinant vaccine production using a virus vector'
- CHEMICAL ABSTRACTS, vol. 98, no. 11, 14. M{rz 1983, Columbus, Ohio, US; abstract no. 84274b, J. STACZEK ET AL. 'Genetic relatedness of the genomes of equine herpesvirus types 1,2, and 3.' Seite 128 ;Spalte 2 ;
- VIROLOGY Bd. 173, Nr. 2, Dezember 1989, NEW YORK, US Seiten 566 - 580 J.M. COLACINO ET AL. 'Physical structure and molecular cloning of equine cytomegalovirus DNA'

## Beschreibung

Die vorliegende Erfindung betrifft equine Herpesviren (EHV), die fremde DNA-Elemente zusätzlich zu den für ihre Replikation notwendigen Genomsequenzen enthalten, sowie ihre Verwendung in Impfstoffen gegen Infektionen.

Bei den erfindungsgemäßen equinen Herpesviren handelt es sich um nicht-virulente oder attenuierte Equine Herpesviren vom Serotyp 2 (EHV-2), die neben den zu ihrer Replikation notwendigen Genomsequenzen mindestens ein fremdes DNA-Element tragen, welches eine Länge von mindestens 20 Nukleotiden aufweist und in einem Fragment inseriert ist, das dem EcoRI-B und/oder -C Fragment des EHV-2 Stammes Thein 400/3, CNCH I-981 entspricht. Herpes- und Influenza-Viren wie auch equine Rhinoviren, Säugerreoviren, equine Adenoviren, Mycoplasmen und Bakterien wie beispielsweise Streptokokken und Corynebakterien stellen ätiologisch die Hauptprobleme innerhalb der infektiösen Atemwegserkrankungen des Pferdes dar. Bei den Allgemeininfektionen kommen dazu Arteritisvirus, Salmonellen, E. coli. Clostridien sowie mit Absiedelung am Intestinum Rota- und Coronaviren. Gegen diese Erreger gilt es wirksame, verträgliche und einfache einzusetzende Impfstoffe zu entwickeln.

Die equinen Herpesviren sind enzootisch in allen Pferdezuchtgebieten der Welt verbreitet und spielen eine dominierende Rolle innerhalb der Infektionskrankheiten der Pferde. Bislang konnten insgesamt vier equine Herpesviren-Serotypen indentifiziert werden.
- EHV-1: (Equines Abortvirus), ein zu den Alpha-Herpesviren zählender Erreger, vormals als EHV-1 Subtyp 1 (Rhinopneumonitisvirus) bezeichnet,
- EHV-2: (Equines Cytomegalie-ähnliches Virus), ein Beta-Herpesvirus,
- EHV-3: (Equines Coitalexanthermvirus), den Alpha-Herpesviren angehörend und
- EHV-4: ebenfalls ein Alpha-Herpesvirus, vormals als EHV-1 Subtyp-2 bezeichnet.

Die equinen Herpesviren verursachen weltweit wirtschaftliche Verluste in der Pferdezucht. Diese entstehen insbesondere durch immer wieder auftretende, seuchenhafte Aborte, Atemwegserkrankungen und zum Teil dramatisch verlaufende Encephalitiden. Von nicht zu unterschätzender wirtschaftlicher Bedeutung sind Aufzuchtverluste, Trainingsausfälle und Leistungseinbußen.

Herpesviren zeigen im allgemeinen eine nur schwach ausgeprägte Immunogenität, die zu einer quantitativ geringen humoralen Immunantwort führt. So resultiert z.B, die respiratorische Verlaufsform nach EHV-4 Infektion häufig in einer serologisch nur schwach ausgeprägten humoralen Immunreaktion. In der Praxis werden derzeit vorwiegend monovalente EHV-1 Lebendimpfstoffe oder inaktivierte EHV-1 Impfstoffe z.T. in Kombination mit heterologen Antigenen eingesetzt. Trotz Einsatz dieser Impfstoffe kommt es immer wieder zu klinischen Erkrankungen durch Infektionen mit Pferdeherpesviren. Dies können Erkrankungen durch Herpesviren der verschiedenen Serotypen monokausal oder im Zusammenspiel mit anderen der genannten Erreger Folgen von Mischungsinfektionen sein (Faktorenerkrankung).

Insgesamt ist die Immunpräventive mit den derzeit einsetzbaren Impfstoffen noch nicht befriedigend. Es ist daher wünschenswert, zu Pferde-Vakzinen zu kommen, die problemlos anzuwenden sind und einen belastbaren Immunschutz gegen möglichst viele Erreger übermitteln.

Bestimmte Vektor-Vakzinen, basierend auf apathogenen Erregern, die neben ihren zur Vermehrung essentiellen Genom-Sequenzen für heterologe Immunogene kodierende Fremd-DNA enthalten, sind bekannt. Es ist auch bekannt, daß in einigen Fällen Antigene pathogener Erreger in avirulente Erreger, also in Vektoren, inseriert wurden.

Alpha- und Gamma-Herpesviren, die Fremd-DNA enthalten, wie beispielsweise Pseudorabies Virus (Aujeszky-Virus) und das Virus der Marek'schen Krankheit und ihre Verwendung in Impfstoffen sind bekannt (PCT Patentanmeldung WO87/4463; WO89/1040). Es ist jedoch nichts bekannt über die Verwendung von equinen Herpesviren, speziell Beta-Herpesviren, wie EHV-2, als Vektoren für Fremd-DNA.

Alpha- und Gamma-Herpesviren unterscheiden sich in ihrem biologischen Verhalten und ihrem strukturellen Aufbau grundsätzlich von den Beta-Herpesviren. Es können also keine Rückschlüsse aus dem Verhalten von Alpha- und Gamma-Herpesviren auf Beta-Herpesviren gezogen werden.

Die aufgeführten Begriffe haben folgende Bedeutung:
- Zur Replikation von EHV notwendige Genomsequenzen sind
   Teile des Gesamt-Genoms von EHV, die zur Vermehrung von EHV, d.h. zur Bildung von Virus-Nachkommen, unverzichtbar sind. Eine Lokalisation einiger Sequenzen liegt nur für wenige Herpesviren und in noch geringerem Maß für EHV vor. Für die Durchführung der vorliegenden Erfindung ist es erforderlich, diese zur Replikation notwendigen Genom-Sequenzen unverändert zu lassen, prüfbar dadurch, ob das veränderte Virus zur Bildung von infektiösen Nach-kommen fähig ist.
- Fremde DNA-Elemente (Fremd-DNA) sind
   DNA-Sequenzen, z.B. Fremd-Gene, die in dem eingesetzten EHV Vektor-Virus ursprünglich nicht vorhanden sind.
   Fremd-DNA wird aus folgenden *Gründen* in das Vektor-Virus inseriert:
   1. zur Expression der Fremd-DNA
   2. zur Inaktivierung von Funktionen von DNA-Sequenzen des Vektor-Virus
   3. zur Markierung des Vektor-Virus

   In Abhängigkeit von diesen Gründen wird unterschiedliche Fremd-DNA inseriert. Soll gemäß (1) Fremd-DNA exprimiert werden, muß die inserierte Fremd-DNA mindestens einen offenen Leserahmen tragen, der für das oder die gewünschten Fremdprotein(e) kodiert. Gegebenenfalls enthält die Fremd-DNA zusätzlich eigene oder fremde Regulatorsequenzen. Die Länge der inserierten Fremd-DNA richtet sich danach, daß die gewünschte Funktion des exprimierten Proteins gewährleistet ist. Im allgemeinen ist die Länge zwischen 1-Nukleotid und 20 kB, bevorzugt zwischen 0,5 und 15 kB.
   Als Beispiele seien genannt, Gene für Immunogene von equinen Herpesviren der Typen 1 bis 4, equine Influenza-Viren, Rhinoviren, Adenoviren, Säugereoviren, Mycoplasmen und Bakterien, wie beispielsweise Streptokokken und Cornebakterien, ferner Arteritisvirus, Rickettsien, Salmonellen, Clostridien, E. coli, Rota- und Coronaviren. Immunogene von equinen Herpesviren der Typen 1 bis 4 können sein die viralen Glykoproteine gB, gC und gD (D.W. Bell, D.B. Boyle, J.M.Whalley (1990) J.Gen.Virol. 71, 1119-1129; p-Guo, S.Goebel, S.Davis, M.E.Perkus, B.Languet, P.Desmettre, G.Allen, E.Paoletti (1989) J.Virol 63, 4189-4198; P.Guo (1990) Gene 87, 249-255; J.M.Whalley, G.R.Robertson, M.A. Scott, G.C.Hudson, C.W.Bell, L.M. Wordworth (1989) J.Gen. Virol. 70, 383-394), wobei diese Bezeichnungen aus der Nomenklatur der HSV-Glykoproteine übernommen wurden. Immunogene der genannten Erreger können sein die viralen Glykoproteine oder Membranproteine der anderen genannten Erreger.
   Sollen gemäß (2)DNA-Sequenzen inaktiviert werden, reicht die Unterbrechung der eigenen DNA-Sequenz des Vektor-Virus. Die maximale Länge der zur Inaktivierung inserierten Fremd-DNA richtet sich nach der Aufnahmekapazität des Vektor-Virus für Fremd-DNA. Die Länge der Fremd-DNA ist bevorzugt zwischen 0,1 und 15 kB.
   Sollen gemäß (3) DNA Sequenzen zur Markierung inseriert werden, richtet sich ihre Länge nach der Nachweismethode zur Identifizierung des markierten Virus. Die Länge der Fremd-DNA ist bevorzugt zwischen 20 Nukleotiden und 15 kB.
- Vektor-Virus ist
   ein EHV-2, das zur Insertion von Fremd-DNA geeignet ist und die inserierte Fremd-DNA in seinem Genom in infizierte Zellen oder Organismen transportieren kann und somit dort die Expression der Fremd-DNA ermöglicht.
- Nicht-virulente EHV sind
   Viren, die nach natürlicher oder experimenteller Infektion von Pferden nicht zur Manifestation von klinischen Symptomen führen.
- Attenuierte EHV sind
   Viren, die durch Veränderung in ihrem Genom vermindert- oder nicht-pathogen bzw. -virulent für Pferde geworden sind.
- Repetitive DNA-Sequenzen sind
   Sequenzen von Nukleotid Bausteinen, die wiederholt hintereinander oder verstreut über das Genom des EHV vorkommen.
- Zur Replikation von EHV nicht notwendige Genom-sequenzen sind
   Teile des Gesamt-Genoms von EHV, die zur Vermehrung von EHV nicht notwendig sind.
   Es kann sich um repetitive DNA-Sequenzen handeln. Dies können auch Sequenzen sein, die, wenn sie nicht wiederholt vorkommen würden, für die Replikation essentiell sein würden.
   Dies können auch DNA-Sequenzen sein, die in ihrer Nukleotid-Sequenz keinen "Offenen Leserahmen (Open Reading Frame)" zur Kodierung von Proteinen zeigen und/oder in ihrer Nukleotid-Sequenz für Proteine kodieren, die für die Virusvermehrung nicht-essentiell sind und/oder in ihrer Nukleotid-Sequenz keine für die Virus- bzw. DNA-Vermehrung relevanten Sequenzen zeigen. In jedem Fall ist nach erfolgter Insertion und/oder Deletion die Prüfung auf Vermehrungsfähigkeit des potentiellen Vektor-Virus erforderlich.
- Deletionen sind
   fehlende DNA-Teilstücke aus dem Genom von EHV.
- Deletionsmutanten sind
   Varianten von EHV, die Deletionen in ihrem Genom enthalten.
- Insertionsmutanten sind
   Varianten von EHV, die in ihren ursprünglichen Genomsequenzen zusätzliche DNA-Sequenzen enthalten. Bevorzugt sind Varianten, in die zusätzliche DNA-Sequenzen experimentell inseriert wurden.
- Genbank ist
   die Gesamtheit der in vermehrungsfähigen Vektoren enthaltenen Fragmente eines Genoms.
   Sie wird durch Fragmentierung des Genoms und Inserierung aller, einzelner oder eines Großteils der Fragmente in vermehrungsfähige Vektoren wie z.B. Plasmide erhalten.
- Genomfragment ist
   ein Teilstück eines Genoms, das isoliert vorliegen oder in einen vermehrungsfähigen Vektor inseriert sein kann.
- Physikalische Genomkarte ist
   die Zuordnung von Restriktionsenzym-Erkennungsstellen einer DNA-Sequenz, die schematisch gestreckt oder zirkular dargestellt ist.
   Physikalische Genomkarten von EHV-2-Stämmen sind bekannt durch "Physical Mapping of a Genome of Equine Herpesvirus 2 (Equine Cytomegalovirus)" Browning, G.F. and Studdert, M.J. (1989), Arch. Virol, 104, 77-86 und Virology 173 (1989), Seite 566-580, J.M. Colacino et al.
   Eine physikalische Genomkarte des EHV-2-Stammes Thein400/3 wird weiter unten beschrieben.
- Geeignete Insertionsstellen sind
   Loci in einem Virus-Genom, die zur Aufnahme von Fremd-DNA geeignet sind.
   Ihre Identifizierung erfolgt z.B. nach folgenden Methoden:
   a) Identifizierung von potentiell nicht-Proteinkodierenden Sequenzen durch Bestimmung der Nukleotid-Sequenz eines viralen DNA-Fragmentes,
   b) Identifizierung von potentiell Proteinkodierenden Regionen, deren Proteine nicht für die Virusvermehrung essentiell sind, durch Bestimmung der Nukleotid-Sequenz eines viralen DNA-Fragmentes
   c) Identifizierung von DNA-Sequenzen, die bei der Virus- bzw. seiner DNA-Vermehrung nicht essentiell sind, durch Bestimmung der Nukleotid-Sequenz eines viralen DNA-Fragmentes,
   d) Identifizierung von repetitiven Sequenzen durch z.B. DNA/DNA-Hybridisierung und gegebenenfalls Nukleotid-Sequenz-Analysen,
   e) Modifikation der Genom-Sequenzen, z.B. durch Nukleotid-Austausch, -Deletion oder-Insertion oder von Kombinationen hieraus,
   f) jede beliebige Kombination aus a), b), c), d) und e),
   und anschließender Überprüfung der Bedeutung der potentiellen Isertionsstelle für die Vermehrungsfähigkeit des potentiellen Vektor-Virus, wobei die identifizierte Insertionsstelle als geeignet zu bezeichnen ist, wenn nach Insertion von Fremd-DNA in diese Stelle die Vermehrung des rekombinanten Vektor-Virus nicht verhindert ist.
- "Offener Leserahmen" ist ein Leseraster (open reading-frame) das durch kein Stoppcodon unterbrochen ist. Gewöhnlich wird eher anhand der DNA-als der RNA-Sequenz überprüft, ob ein offenes Leseraster vorliegt. Crick, F.H.C., L. Barnett, S. Brenner, and R.J. Watts Tobin, 1961. "General Nature of the Genetic Code for Proteins" Nature 192: 1227-1232.
- Inaktivierung durch Inserierung bedeutet,
   daß die inserierte Fremd-DNA die Expression oder Funktion EHV-eigener Genom-Sequenzen verhindert.
- Markierung durch Inserierung bedeutet,
   daß die inserierte Fremd-DNA die spätere Identifizierung des veränderten EHV ermöglicht.
- Plasmide, die einsetzbar sind,
   sind z.B. bakterielle Plasmide. Dies sind ringförmige, extrachromosomale DNA-Sequenzen, die sich in Bakterien vermehren können. Solche sind bekannt aus "Molecular Cloning", A Laboratory Manual, 2nd Edition 1989, ed. J. Sambrook, E.F. Fritsch und T. Maniatis, Cold Spring Harbor Laboratory Press, z.B. besonders geeignet sind pBR322, pAT153, pUC18, pUC19, pACYC184. Der Umgang mit diesen Plasmiden erfolgt gemäß den in der genannten Literatur angegebenen Methoden.
- Andere Vektoren, die einsetzbar sind,
   sind z.B. Bakteriophagen, z.B. Bakteriophage Lambda.
- Shuttle-Vektoren sind
   Plasmide, insbesondere bakterielle Plasmide, die die zu inserierende Fremd-DNA, flankiert von DNA-Sequenzen des Vektor-Virus, beinhalten. Ihre Herstellung, Vermehrung und Verwendung wird weiter unten beschrieben.
- Regulatorsequenzen sind
   DNA-Sequenzen, die die Expression von Genen beeinflussen. Solche sind bekannt aus "Molecular Biology of the Gene", Watson, J.D.; Hopkins, N.H.; Roberts, J.W.; Steitz, J.A. and Weiner, A.M. 1987), The Benjamin/Cummings Publishing Company, Menlo Park. Bevorzugt seien genannt Promotoren, wie z.B. der Rous Sarcoma Virus-Promotor (Gorman et al. (1983) Science 221, 551-553), der EHV-1-gB-Promotor (J. Gen.Virol. (1989), 70 383-394, Identification and Nucleotide Sequence of a Gene in Equine Herpesvirus 1, Analogues to the Herpes Simplex Virus gene Encoding the Major Envelope Glycoprotein gB, by J. M. Whalley, G.R. Robertson, A. Scott, Grant C. Hudson, C. W. Bell and L.M. Woodworth, sowie Journal of General Virology (1990), 71 1119-1129, Transcript analysis of the equine herpesvirus 1 glycoprotein B gene homologue and its expression by a recombinant vaccinia virus, C.W. Bell, D.B. Boyle and J. M. Whalley) oder der HSV-1-Thymidinkinase-Promotor (McKnight (1980) Nucleic Acids Res. 8 5949-5963).
- Transfektion ist
   das Einbringen von DNA-Sequenzen z.B. Fremd-DNA, die in Shuttle-Vektoren enthalten sind oder gereinigte EHV-DNA, in Zellen, die zur Virusvermehrung geeignet sind, mit dem Ziel Virus-Rekombinanten zu induzieren, die fremde DNA-Sequenzen enthalten. Die zur Virusvermehrung geeigneten Zellen können auch vor oder nach der Transfektion mit dem Vektorvirus infiziert werden, um die Virus-Rekombinanten die Fremd-DNA enthalten, zu erzeugen.
- Co-Transfektion ist
   das simultane Einbringen von mindestens zwei verschiedenen DNA-Sequenzen in Zellen, die zur Virusvermehrung geeignet sind, mit dem Ziel Virus-Rekombinanten zu induzieren, die fremde DNA-Sequenzen enthalten. Bei den verschiedenen DNA-Sequenzen handelt es sich einerseits um Fremd-DNA, die in Shuttle-Vektoren eingebaut sein kann und andererseits um gereinigtes Gesamt-Genom des Vektorvirus.
   Arbeitsmethoden zur Durchführung der Transfektion oder Co-Transfektion sind bekannt aus "Methods in Virology Vol. VI" (1977), ed. K. Maramorosch, H. Koprowski, Academic Press New York, San Francisco, London. Bevorzugt wird die Methode der sogenannten Calzium-Phosphat-Technik ("Methods in Virology Vol. VI" (1977), ed. K. Maramorosch, H. Koprowski, Academic Press New York, San Francisco, London).
- Antigene sind
   Proteine oder Peptide deren Expression die Fremd-DNA im Vektorvirus und/oder durch das Genom des Vektor-virus ermöglicht wird.
- Immunogene sind
   kurzkettige Peptide oder Proteine, die im immunisierten Organismus zu einem homologen Schutz vor den klinischen Folgen der Infektion führen und deren Expression durch Fremd-DNA und/oder Genom des Vektorvirus ermöglicht wird.
- Translationseinheiten sind translatierte Aminosäuresequenzen, Peptide oder Proteine.
- Epitop
   ist eine spezifische Bindungstelle für Antikörper auf Basis einer Aminosäuresequenz.

Die Herstellung der erfindungsgemäßen equinen Herpesviren (EHV) erfolgt beispielsweise wie nachstehend angegeben:
1. Auswahl eines geeigneten EHV-2 Stammes.
2. Etablierung der viralen Genbank des geeigneten EHV-Stammes.
3. Konstruktion der physikalischen Karte des viralen Genoms des geeigneten EHV-Stammes.
4. Identifizierung von potentiellen Insertionsstellen für Fremd-DNA im Genom des geeigneten EHV-Stammes.
5. Konstruktion eines Shuttle-Vektors zur Übertragung fremder genetischer Elemente in das Genom des geeigneten EHV-Stammes.
6. Konstruktion eines rekombinanten EHV-Virus, das fremde DNA enthält und z.B. bei seiner Replikation Fremdprotein exprimiert.

### Zu 1.

### Auswahl eines geeigneten EHV Stammes

Grundsätzlich sind alle EHV-2-Stämme für den Einsatz im erfindungsgemäßen Verfahren geeignet.

Besonders bevorzugte Stämme vom Typ 2 sind solche, die sich zu Titern ≧ 10⁵ PFu (Plaque Forming Unit)/ml in einer Gewebekultur vermehren lassen und aus dem Medium der infizierten Zellen als extrazelluläres, infektiöses Virus rein darstellen lassen.

Als besonders geeignet erwies sich der EHV-2 Stamm Thein-400/3, hinterlegt nach dem Budapester Vertrag am 24.7,1990 bei Institut Pasteur, C.N.C.M. unter der Reg. Nr. I-981, sowie seine Varianten und Mutanten.

Die Vermehrung der Viren erfolgt in üblicher Weise in Gewebekulturen animaler Zellen, z.B. in Pferde-Zellen, Affen-Zellen oder Kaninchen-Zellen, bevorzugt in Pferdehaut-Zellen wie der permanenten Pferde-Haut-Zelle ED (ATCC CCL 57 oder deren Abkömmlingen) oder Kaninchennieren-Zellen wie in der Kaninchennieren-Zelle RK-13 (ATCC CCL37 oder deren Abkömmlingen) oder in Affennieren-Zellen.

Die Vermehrung erfolgt in an sich bekannter Weise in stationären, Roller- oder Carrier-Kulturen in Form von geschlossenen Zellverbänden oder in Suspensionskulturen. Als Vermehrungsmedien für die Zellen werden eingesetzt alle an sich bekannten Zellkulturmedien z.B. beschrieben in Produktkatalog der Fa. Flow Laboratoris GmbH Post 1249, 5309 Meckenheim, wie insbesondere das Minimal Essential Medium (MEM), das als wesentliche Bestandteile Aminosäuren, Vitamine, Salze und Kohlenhydrate enthält, komplettiert mit Puffersubstanzen wie z.B. Natrium-Bicarbonat oder (Hydroxyethylpiperazin-N-2-ethansulfonsäure (Herpes) und gegebenenfalls Tierseren, wie z.B. Seren von Rindern, Pferden bzw. deren Foeten. Besonders bevorzugt wird der Einsatz von foetalem Kälberserum in einer Konzentration von 1-30 Vol-%, vorzugsweise 2-10 vol-%.

Die zur Vermehrung der Viren dienenden Zellen und Zellrasen werden in üblicher Weise nahezu bis zur Konfluenz oder bis zu optimaler Zelldichte vermehrt. Vor ihrer Infektion mit Viren wird bevorzugt das Zellvermehrungsmedium entfernt und die Zellen bevorzugt mit Virusvermehrungsmedium gewaschen. Als Virusvermehrungsmedien werden eingesetzt, alle an sich bekannten Zellkulturmedien, wie insbesondere das oben genannte MEM. Danach wird mit einer Virussuspension infiziert. In der Virussuspension liegt das Virus im Virusvermehrungsmedium derart verdünnt vor, daß mit einer MOI (= multiplicity of infection entspricht infektiöse Viruspartikel auf vorhandene Zellen) von 0,01-50, bevorzugt 0,10-10 infiziert wird.

Die Vermehrung der Viren erfolgt mit oder ohne Zusatz von Tierseren. Für den Fall, daß Serum eingesetzt wird, wird dieses zum Vermehrungsmedium in einer Konzentration von 1-30 Vol.-%, vorzugsweise 2-10 Vol-% zugegeben.

Infektion und Virus-Vermehrung erfolgt bei Temperaturen zwischen Raumtemperatur und 40°C, bevorzugt zwischen 32 und 39°C, besonders bevorzugt bei 37°C über mehrere Tage, bevorzugt bis zur vollständigen Z_{erstörung} der infizierten Zellen.

Das virushaltige Medium der infizierten Zellen kann weiter aufgearbeitet werden, z.B. durch Entfernung der Zelltrümmer mittels Filtration mit Porengrößen von z.B. 0,1-0,45 µm und/oder Zentrifugation bis zu 10.000 g.

Filtrat oder Zentrifugationsüberstand können zur Virusanreicherung und -reinigung verwendet werden. Dazu werden Filtrat oder Überstand einer hochtourigen Zentrifugation bis zur Sedimentation der Viruspartikel unterworfen. Gegebenenfalls können weitere Reinigungsschritte durch z.B. Zentrifugation in einem Dichtegradienten angeschlossen werden.

### Zu 2.

### Etablierung der viralen Genbank

Aus dem Genom des EHV-2, z.B. des EHV-2 Stammes Thein400/3,wird eine definiert Genbank nach den aus "Molecular Cloning", A Laboratoy Manual, 2nd Edition 1989, ed. J. Sambrook, E.F. Fritsch und T. Maniatis, Cold Spring Harbor Laboratory Press an sich bekannten Methoden, etabliert.

Ausgehend von dem gemäß (oben) hergestellten und gereinigten Virus wird das Genom isoliert und gereinigt.

Die Extraktion von nativer viraler DNA erfolgt bevorzugt über die Behandlung der gereinigten Virionen mit wäßrigen Lösungen von Detergentien und Proteasen.

Als Detergentien seien genannt anionische, kationische, amphotere, nicht-ionische Detergentien. Bevorzugt werden ionische Detergentien eingesetzt. Besonders bevorzugt werden Natriumdodecylsulfat, Natriumlaurylsulfat.

Als Proteasen seien genannt, alle Proteasen die in Gegenwart von Detergens arbeiten, wie z.B. Proteinase K. und Pronase. Bevorzugt sei genannt Proteinase K.

Detergentien werden in Konzentrationen von 0,1 - 10 Vol-% eingesetzt, bevorzugt sind 0,5-3 Vol.-%.

Proteasen werden in Konzentrationen von 0,01 - 10 mg/ml Viruslysat eingesetzt, bevorzugt sind 0,05 - 0,5 mg/ml Viruslysat.

Es wird bevorzugt in wäßriger gepufferter Lösung in Gegenwart von DNase Inhibitoren gearbeitet. Als Puffersubstanzen seien genannt: Salze schwacher Säuren mit starken Basen wie z.B. Tris(hydroxymethylaminomethan), Salze starker Säuren mit schwachen Basen wie z.B. primäre Phosphate oder Gemische derselben.

Bevorzugt sei das folgende Puffersystem genannt: Tris(hydroxymethylaminomethan).

Die Puffersubstanzen oder Puffersysteme werden in Konzentrationen eingesetzt, die pH-Werte gewährleisten, bei denen die DNA nicht denaturiert. Bevorzugt sind pH-Werte von 5-9, besonders bevorzugt 6-8,5, ganz besonders bevorzugt 7-8, insbesondere sei Arbeiten im neutralen Bereich genannt.

DNase-Inhibitoren sind z.B. Ethylendiamintetraessigsäure in Konzentrationen von 0,1 - 10 Mmol, bevorzugt ist ca. 1 Mmolar.

Anschließend werden die lipophilen Bestandteile des Viruslysats extrahiert. Als Extraktionsmittel dienen Lösungsmittel wie Phenol, Chloroform, Isoamylalkohol oder deren Gemische. Bevorzugt wird zunächst ein Gemisch aus Phenol und Chloroform/Isoamylalkohol eingesetzt, wobei die Extraktion in einer oder mehreren Stufen erfolgt.

In der letzten Stufe der Extraktion wird bevorzugt Chloroform/Isoamylalkohol eingesetzt. Alternativ kann zunächst Phenol und anschließend Chloroform/Isoamylalkohol eingesetzt werden.

Weitere Methoden zur Isolierung der Viren DNA sind z.B. Zentrifugation eines Viruslysats in einem CsCl -Dichtegradienten oder in der Gelelektrophorese (Sharp et al. Biochem. 1973 (12) S. 3055-3063).

Die Extraktion von Nukleinsäuren wird in "Molecular Cloning", A Laboratory Manual, 2nd Edition 1989, ed J.

Sambrook, E.F. Fritsch und T. Maniatis, Cold Spring Harbor Laboratory Press beschrieben.

Die so extrahierte DNA wird bevorzugt aus der wäßrigen Lösung mit z.B. Alkohol, bevorzugt mit Ethanol oder Isopropanol und unter Zusatz von monovalenten Salzen wie z.B. Alkalichloride oder -acetate, bevorzugt Lithiumchlorid, Natriumchlorid oder Natriumacetat, Kaliumacetat, ausgefällt.

Die Konzentration an Alkohol liegt hierbei zwischen 40 und 100 Vol-%, bevorzugt zwischen 60 und 80 Vol-%, besonders bevorzugt bei ca. 70 Vol-%.

Die Chlorid- oder Acetatkonzentration liegt zwischen 0,01 oder 1 molar, bevorzugt zwischen 0,1 und 0,8 molar, Wird LiCl eingesetzt, liegt seine Konzentration zwischen 0,1 und molar, bevorzugt zwischen 0,4 und 0,8 molar.

Methoden zur Präzipitation von Nucleinsäuren sind in "Molecular Cloning" loc. cit. ausführlich beschrieben. Die präzipitierte DNA wird durch z.B. Zentrifugation aus der wäßrigen Suspension isoliert, vorzugsweise mit Alkohol, z.B. 70 Vol-% Ethanol gewaschen und schließlich in wäßriger Pufferlösung resolubilisiert.

Als Puffersubstanz sei genannt Tris(hydroxymethyl)aminomethan in Konzentrationen von 1- 100 Mmol, bevorzugt wird 10-50 Mmol. Bevorzugte pH-Werte sind 6-8,5, besonders bevorzugt 7-8.

Als weitere Zusätze seien genannt, z.B. EDTA (Ethylendiaminotetraessigsäure) in Konzentrationen von 0,1-10 Mmolar, bevorzugt 1-10 Mmolar.

Alternativ kann die präzipitierte DNA auch in 0,01 oder 0,1 x SSC-Puffer (Molecular Cloning) loc. cit. oder im Ammoniumcarbonatpuffer resolubilisiert werden.

Die so gereinigte virale DNA wird mit Restriktonsendonuklease nach Vorschrift der Hersteller behandelt. Geeignete Restriktionsendonucleasen sind solche die mindestens eine für sie spezifische Schnittstelle auf dem Virusgenom erkennen. Restriktionsendonukleasen (Restriktionsenzyme) sind z.B. EcoRI, BamHI, SalI, HindIII, PstI, XbaI, AflIII oder BspMII. Die resultierenden DNA-Fragmente werden nach den bei "Molecular Cloning", A Laboratory Manual, 2nd Edition 1989, ed. J. Sambrook, E.F. Fritsch und T. Maniatis, Cold Spring Harbor Laboratory Press beschriebenen Methoden in die korrespondierenden Erkennungssequenzen von bakteriallen Plasmid-Vektoren, Phagen oder Cosmiden molekular kloniert. Besonders bevorzugt werden für niedermolekulare DNA-Fragmente (bis ca. 15 kbp) Plasmid-Vektoren, für DNA-Fragmente ab ca. 15 kbp bis zu ca. 45 kbp Cosmide.

### Zu 3.

### Konstruktion der physikalischen Karte des Genoms

Anschließend wird die physikalische Karte des viralen Genoms, z.B. des EHV-2 Stammes Thein-400/3, anhand an sich bekannter Methoden, z.B. DNA/DNA-Hybridisierung, partielle Restriktion mit Restriktionsendonukleasen oder zweifachen Restriktionen mit Restriktionsendonukleasen (Double Digest), bevorzugt unter Anwendung der viralen Genbank, und DNA-DNA-Hybridisierung konstruiert.

Die Methoden sind bekannt aus "Molecular Cloning" loc. cit, oder "A Practical Guide to Molecular Cloning 2nd, ed. B. Perbal, Wiley Interscience 1988.

### Zu 4.

Die Identifizierung geeigneter Insertionsstellen erfolgt auf den Virus-Genom-Fragmenten Eco RI-B und/oder -C z.B. durch
a) Identifizierung von DNA-Sequenzen, die nicht für Proteine kodieren. Z.B. durch Bestimmung der Nukleotid-Sequenz des jeweiligen Virus-Genom-Fragments, wobei Nukleotid-Sequenzen gesucht werden, die Regionen enthalten die nicht für Proteine kodieren. Die in diesen Regionen vorliegenden Erkennungsstellen für Restriktionsenzyme stellen potentielle Insertionsstellen dar. Um festzustellen, ob eine potentielle Insertionsstelle eine geeignete Insertionsstelle im Gesamtgenom von EHV ist, muß Fremd-DNA in das Fragment inseriert werden und das Fragment mit dem Insert in das Virusgenom eingebaut werden. Anschließend wird das Fremd-DNA enthaltende rekombinante Virus auf Vermehrungsfähigkeit geprüft. Vermehrt sich das Fremd-DNA enthaltende rekombinante Virus, ist die oben identifizierte Erkennungsstelle als Insertionsstelle geeignet.
b) Identifizierung von DNA-Sequenzen, die für Proteine kodieren, welche für die Virusvermehrung nicht essentiell sind, z.B. durch Bestimmung der Nukleotidsequenz und Identifizierung von "offenen Leserahmen" für nicht-essentielle Proteine. Als nicht-essentielle Proteine werden Proteine angesehen, die von EHV und anderen Herpesviren als EHV bekannt sind und sich dort als für die Vermehrung nicht-essentiell herausgestellt haben. Man geht davon aus, daß solche Proteine, falls sie bei EHV auftreten, auch bei EHV nicht essentiell sind. Es wird daher die oben erwähnte Nukleotidsequenz des EHV-Genomfragments darauf untersucht, ob sie einen "offenen Leserahmen" für eines der bei anderen Herpesviren bekannten, nicht-essentiellen Proteins enthält. Um festzustellen, ob diese potentiellen Insertionsstellen geeignete Insertionsstellen im Gesamtgenom von EHV sind, muß Fremd-DNA in das Fragment inseriert werden und das Fragment mit dem Insert in das Virusgenom eingebaut werden. Anschließend wird das Fremd-DNA enthaltende rekombinante Virus auf Vermehrungsfähigkeit geprüft. Vermehrt sich das Fremd-DNA enthaltende rekombinante Virus, ist die oben identifizierte Erkennungsstelle als Insertionsstelle geeignet.
c) Identifizierung von DNA-Sequenzen die für die DNA-bzw. Virusvermehrung nicht-essentiell sind. Als solche Sequenzen werden Sequenzen angesehen, von denen bei EHV und/oder anderen Herpesviren als EHV bekannt ist, daß sie nicht essentiell sind. Dies erfolgt z.B. durch vergleichende Nukleotidsequenzanalyse des EHV-Genomfragments. Um festzustellen, ob diese potentiellen Insertionsstellen geeignete Insertionsstellen im Gesamtgenom von EHV sind, muß Fremd-DNA in das Fragment inseriert werden und das Fragment mit dem Insert in das Virusgenom eingebaut werden. Anschließend wird das Fremd-DNA enthaltende rekombinante Virus auf Vermehrungsfähigkeit geprüft, Vermehrt sich das Fremd-DNA enthaltende rekombinante Virus, ist die oben identifizierte Erkennungsstelle als Insertionsstelle geeignet.
d) Identifizierung von repetitiven Sequenzen durch z.B. DNA/DNA-Hybridisierung und anschließende Identifizierung von Erkennungssequenzen von Restriktionsenzymen in diesen repetitiven Sequenzen, z.B. durch Nukleotidsequenzanalyse. Die angegebene DNA/DNA-Hybridisierung erfolgt, indem z.B. in Vektoren inserierte Genom-Fragmente aus der Genbank mit dem durch Restriktionsenzyme fragmentierten Genom des EHV hybridisiert werden. Die Erstellung der Genbank und die Fragmentierung muß dabei mit demselben Restriktionsenzym erfolgt sein. Die in Vektoren inserierten Genom-Fragmente, die mit mehr als einem Fragment des Gesamtgenoms hybridisieren, enthalten eine oder mehrere repetitive Sequenzen. Um die repetitive Sequenz auf dem Genom-Fragment zu lokalisieren, wird die Nukleotidsequenz bestimmt und miteinander verglichen. Identische Sequenzen auf dem Fragment sind repetitiv. Um festzustellen, ob eine potentielle Insertionsstelle eine geeignete Insertionsstelle im Gesamtgenom von EHV ist, muß Fremd-DNA in das Fragment inseriert werden und das Fragment mit dem Insert in das Virusgenom eingebaut werden. Anschließend wird das Fremd-DNA enthaltende rekombinante Virus auf Vermehrungsfähigkeit geprüft, Vermehrt sich das Fremd-DNA enthaltende rekombinante Virus, ist die oben identifizierte Erkennungsstelle als Insertionsstelle geeignet.
e) Modifikation der Genom-Sequenzen, z.B, durch Nukleotid-Austausch, -Deletion oder -Insertion oder Kombinationen hieraus,
f) jede beliebige Kombination aus a), b), c), d) und e).

Bevorzugt werden repetitive Sequenzen über DNA/DNA-Hybridisierung lokalisiert und über Nukleotid-Sequenz-Analysen näher charakterisiert.

Die Insertionsstellen müssen zur Aufnahme von Fremd-DNA Sequenzen einer Länge geeignet sein, die sich nach ihren oben genannten Aufgaben richtet.

Bevorzugt sind Insertionsstellen, in die Fremd-DNA einer Länge bis zu 15 kB inseriert werden kann.

Die Kapazität zur Aufnahme von Fremd-DNA kann durch Setzen von Deletionen im Genom des Virus vergrößert werden. Die Deletionen bewegen sich in einer Größe von 1 Nukleotid bis 15 kB, bevorzugt von 1 - 10 kB.

### Zu 5.

### Konstruktion eines Shuttle-Vektors zur Übertragung fremder genetischer Elemente.

Shuttle-Vektoren werden zur Transferierung von Fremd-DNA in das Vektor-Virus hergestellt, indem die Fremd-DNA mit DNA-Sequenzen des Vektor-Virus flankiert wird. Die Konstruktion erfolgt in folgenden Stufen:
a) Isolierung der die Insertionsstelle tragenden DNA-Sequenz aus dem viralen Genom mit Restriktionsendonukleasen und ihre Insertion in ein Plasmid oder einen Phagenvektor.
   Hierzu wird das EHV wie unter 1 (Auswahl eines geeigneten EHV Stammes) angegeben vermehrt und als extrazelluläres Viruspartikel gereinigt. Aus diesem Viruspartikel wird das Virusgenom isoliert und fragmentiert wie unter 2 (Etablierung der viralen Genbank) angegeben.
   Die dabei entstandenen DNA-Fragmente werden nach ihrer z.B. elektrophoretischen Auftrennung aus dem Trenngel (z.B. Agarosegel) isoliert und das Fragment, das die identifizierte Insertionsstelle trägt in ein Plasmid oder einen Phagenvektor inseriert.
   Als Methoden zur Auftrennung der DNA Fragmente kommen elektrophoretische und chromatographische Verfahren in Frage.

Bei den chromatographischen Verfahren sei genannt Gelfiltration.
Bei den elektrophoretischen Verfahren seien als Träger genannt Agarose oder Polyacrylamid. Als Elektrophoresepuffer seien genannt z.B. Ethylendiamintetraessigsäure, Phosphat/Puffer (EPP) Tris(hydroxymethyl)aminomethan-borat-ethylendiamintetraessigsäure-Puffer (TBE) der wie folgt zusammengesetzt ist:
- Tris: 10-100 mM, bevorzugt 40-90 mM, besonders bevorzugt 80-90 mM,
- Borsäure: 10-100 mM, bevorzugt 40-90 mM, besonders bevorzugt 80-90 mM,
- EDTA: 1-10 mM, bevorzugt 1-2,5 mM
- pH: 7-9, bevorzugt 8-8,5
oder
Tris-(hydroxymethyl)aminomethan-Acetat-Ethylendiamintetraessigsäure-Puffer (TAE) der wie folgt zusammengesetzt ist:
- Tris: 10-100 mM, bevorzugt 30-90 mM, besonders bevorzugt 40 mM,
- Natrium-Acetat: 1-100 mM, bevorzugt 5-50 mM,
- EDTA: 1-10 mM, bevorzugt 1-2,5
- pH: 7-9, bevorzugt 7,5-8,5.

Eine detaillierte Aufstellung und Beschreibung von Elektrophoresepuffern ist in
   - Current Protocols in Molecular Biology 1987-1988, Verlag Wiley-Interscience, 1987
   - A Practical Guide to Molecular Cloning, B, Perbal, 2^{nd} edition Verlag Wiley-Interscience, 1988
   - Molecular Cloning, loc. cit.
   - Virologische Arbeitsmethoden, Band III Gustav Fischer Verlag, 1989

   beschrieben.
   Die Durchführung des Verfahrens ist beschrieben in "Molecular Cloning" loc cit, oder in Virolog. Arbeitsmethoden Band 3.
   Die Isolierung des DNA-Fragments mit der Insertionsstelle erfolgt aus dem Träger z.B. durch Elektroelution des das Fragment enthaltenden Trägerbereichs. Alternativ durch low-melting-Agaroseverfahren (Molecular cloning loc, cit.) oder durch Adsorption des DNA-Fragments an Glasoberflächen (Gene-clean^{®} Methode).
   Zur Insertion des DNA-Fragments werden doppelsträngige Plasmid oder Phagenvektor DNA-Moleküle mit Restriktionsenzymen behandelt, so daß für die Inserierung geeignete Enden entstehen.
   Als Plasmide dienen z.B. pAT153, pACYC184, pUC18/19, pBR322, pSP64/65.
   Als Phagenvektoren dienen Lambdaphagenvarianten wie z B. -ZAP, -gt10/11 oder Phage M13mp18/19.
   Die einsetzbaren Restriktionsenzyme sind an sich bekannt z.B. aus Gene Bnd 92 (1989) Elsevier Science Publishers BV Amsterdam.
   Das mit Restriktionsenzym behandelte Plasmid oder der Phagenvektor wird mit einem Überschuß des zu inserierenden DNA-Fragment z.B. etwa im Verhältnis 5 zu 1 gemischt und mit DNA-Ligasen behandelt um das DNA-Fragment End-zu-End in den Vektor kovalent zu binden.
   Ligasen sind Enzyme, die in der Lage sind zwei DNA-Moleküle über 3'-OH-5'-Reste zu verbinden.
   Der Ligierungsansatz wird zur Vermehrung der Plasmide in pro- oder eukaryontische Zellen, bevorzugt in Bakterien eingebracht und diese vermehrt.
   Als Bakterien dienen z.B. Escherichia coli Stamm K-12 und seine Derivate z.B. K 12-600 (Molecular cloning loc. cit.).
   Die Vorbereitung des Ligierungsansatzes und der Bakterienkultur erfolgt in an sich bekannter Weise wie in Molecular cloning loc. cit, beschrieben.
   Die Bakterien, die Plasmide mit inserierter Fremd-DNA enthalten, werden selektiert.
   Alternativ kann ein gemäß 2 (oben) bereits in einen Vektor inseriertes EHV-DNA Fragment, das nach 4 (oben) eine identifizierte Insertionsstelle enthält, als Ausgangsmaterial für die Herstellung des Shuttle Vektors eingesetzt werden.
b) Gegebenenfalls werden sogenannte "Polylinker" in die identifizierte Insertionsstelle inseriert. Hierzu wird das EHV DNA-Fragment mit der identifizierten Insertionsstelle mit einem Restriktionsenzym, das das Fragment nur an einer Stelle öffnet,
   behandelt. Das so geöffnete Fragment wird mit einem Polylinker und Ligase inkubiert, um gezielt andere Restriktionsenzymerkennungsstellen zu inserieren.
   Polylinker sind DNA-Sequenzen die mindestens zwei Restriktionsenzymerkennungsstellen hintereinandergeschaltet tragen.
   Als Ligase sei z.B. genannt T4-DNA-Ligase.
   Der Polylinker wird entweder in das freie DNA-Fragment oder in das in Plasmiden oder Phagenvektoren eingebaute DNA-Fragment inseriert.
   Wird der Polylinker in ein freies DNA-Fragment inseriert, wird das Polylinker enthaltende Fragment anschließend in Plasmide inseriert, die Plasmide in pro- oder eukaryontischen Zellen bevorzugt Bakterien, vermehrt und diese selektiert.
   Wird der Polylinker in DNA-Fragmente inseriert die in Plasmiden enthalten sind, werden die dabei erhaltenen Plasmide in pro- oder eukaryontischen Zellen bevorzugt Bakterien vermehrt und selektiert.
c) Deletion von Teilsequenzen des DNA-Fragments mit der identifizierten Insertionsstelle.
   Das DNA-Fragment wird dazu mit Restriktionsenzym behandelt und die entstandenen Fragmente aufgetrennt. Die Auftrennung erfolgt nach den oben beschriebenen Methoden.
d) Inserierung der Fremd-DNA in die Insertionsstelle.
   Das EHV-DNA-Fragment mit der identifizierten Insertionsstelle wird entweder in freier Form oder in ein Plasmid inseriert mit einem oder mehreren Restriktionsenzymen behandelt, die an der Insertionsstelle bzw. im inserierten Polylinker das Fragment auftrennen.
   Die Fremd-DNA wird nach den an sich bekannten Methoden der sticky-end oder blunt-end Ligation mittels Ligasen in die Erkennungsstelle inseriert. Liegt das EHV-Fragment inseriert, z.B. in einem Plasmid, vor wird der Inkubationsansatz nach der Ligasereaktion in pro- oder eukaryontische Zellen, bevorzugt Bakterien, eingebracht und diese vermehrt und selektiert.
   Liegt das EHV-DNA-Fragment vor der Inserierung mit Fremd-DNA in freier Form vor, wird das DNA-Fragment mit der Insertion in ein Plasmid inseriert, in pro- oder eukaryontische Zellen, bevorzugt Bakterien eingebracht (Transformation), diese vermehrt und selektiert.
   Die oben erwähnten Methoden, die zur Herstellung des Shuttle-Vektors angewendet werden, sind im Detail in "Molecular Cloning", A Laboratory Manual, 2nd Edition 1989, ec. J. Sambrook, E.F. Fritsch und T. Maniatis, Cold Spring Harbor Laboratory Press beschrieben.

### Zu 6.

### Insertion von Fremd-DNA in das Vektor-Virus-Genom

Für den Einbau des fremden genetischen Elements in das Vektor-Virus werden folgende Verfahren angewandt
a) Cotransfektion der Shuttle-Vektor-DNA und der nativen DNA des Vektor-Virus in geeignete Wirtszellen,
b) Transfektion der Shuttle-Vektor-DNA und Infektion mit dem Vektor-Virus in geeignete Wirtszellen,
c) Infektion mit dem Vektor-Virus und Transfektion mit der Shuttle-Vektor-DNA in geeignete Wirtszellen.

Die Methoden der hier geeigneten Verfahren sind beschrieben in "Methods in Virology Vol. VI" (1977), ed. K. Maramorosch, H. Koprowski, Academic Press New York, San Francisco, London. Bevorzugt wird die Methode der sogenannten Calcium-Phosphat-Technik ("Methods in Virology Vol. VI" (1977), ed. K. Maramorosch, H. Koprowski, Academic Press New York, San Francisco, London), Bevorzugt wird das Verfahren a) eingesetzt. Hierzu sind folgende Schritte notwendig:
1. Die nach den vorne beschriebenen Verfahren erhaltenen transformierten Zellen, die Shuttle-Vektoren enthalten, werden vermehrt und die Shuttle-Vektoren in an sich bekannter Weise aus den Zellen isoliert und weiter gereinigt. Die Reinigung erfolgt z.B. durch eine isopyknische Zentrifugation im Dichtegradienten von z.B. CsCl.
   Das Vektor-Virus wird vermehrt und gereinigt. Das virale Genom wird extrahiert und weiter gereinigt. Die Reinigung erfolgt z.B. durch eine isopyknische Zentrifugation im Dichtegradienten von z.B. CsCl.
2. Zur Cotransfektion kann die Shuttle-Vektor-DNA zirkular oder linearisiert eingesetzt werden. Bevorzugt wird die linearisierte Form eingesetzt.
   Die Linearisierung erfolgt beispielsweise durch die Behandlung der in 1. gereinigten DNA des Shuttle-Vektors durch Behandlung mit Restriktionsendonukleasen. Bevorzugt werden Restriktionsendonukleasen, die es erlauben, daß die Fremd-DNA in ihrer Gesamtheit isoliert werden kann. Grundsätzlich kommem hierfür alle Restriktionsendonukleasen in Frage, die keine Erkennungssequenz in der Fremd-DNA haben.
3. Die Virus-Vektor-DNA und die Shuttle-Vektor-DNA werden gemischt im Verhältnis 0,01 bis 0,1 x 10⁻¹² molar an Vektor-Virus-DNA zu 1 bis 3 x 10⁻¹² molar an Shuttle-Vektor-DNA. Am besten geeignet ist das Molekularverhaltnis Vektor-Virus-DNA : Fremd-DNA-Insert im Shuttle-Vektor von 1 : 300
4. Nach dem Mischen der DNA wird diese mit z.B. Calcium-Phosphat copräzipitiert und auf geeignete Zellen übertragen (Cotransfektion) vgl. Molecular cloning loc. cit.
   Geeignete Zellen sind animale Zellen, bevorzugt Säugerzellen, z.B. Pferde- oder Kaninchenzellen, besonders bevorzugt ist die permanente Pferde-Haut-Zellinie ED (z.B. ATCC CCL57 oder deren Abkömmlinge.
   Die gemischte DNA kann auch mit anderen Methoden in die Zellen eingebracht werden.
   Genannt seien die an sich bekannten Methoden mit DEAE-Dextran, oder Liposomen (z.B. Lipofectin^{®}) oder Elektroporation.
5. Die Zellen werden nach den vorne (Auswahl eines geeigneten EHV-Stammes) beschriebenen Methoden kultiviert.
6. Bei Auftreten eines cytopathischen Effektes wird das Kulturmedium entfernt, gegebenenfalls durch Zentrifugation oder Filtration von Zell-Trümmern befreit und gegebenenfalls gelagert sowie nach den herkömmlichen Methoden der Einzel-Plaque-Reinigung von Viren aufgearbeitet.
7. Die Selektion von rekombinanten Vektor-Viren die Fremd-DNA enthalten, erfolgt in Abhängigkeit von der inserierten Fremd-DNA z.B. durch:
   a) Expression der Fremd-DNA mit Hilfe der rekombinanten Vektor-Viren
   b) Nachweis des Vorhandenseins der Fremd-DNA, z.B. durch DNA/DNA-Hybridisierungen.
   zu a)
   Die Expression von Fremd-DNA kann auf RNA- oder Protein-Ebene erfolgen.
   Die Expression der Fremd-DNA auf Protein-Ebene kann nachgewiesen werden durch z.B. Infektion von Zellen mit dem Vektor-Virus und anschließender Immunfluoreszenzanalyse mit Antikörpern gegen das von der Fremd-DNA kodierte Protein oder durch Immunpräzipitation mit Antikörpern gegen das von der Fremd-DNA kodierte Protein aus den Lysaten infizierter Zellen.
   Die Expression der Fremd-DNA auf RNA-Ebene kann nachgewiesen werden durch Hybridisierung der RNA aus mit Vektor-Virus infizierten Zellen mit DNA, die zumindest in Teilen identisch ist mit der inserierten Fremd-DNA.
   zu b.
   Hierzu kann die DNA aus dem fraglichen Virus gewonnen und mit DNA, die zumindest in Teilen mit der inserierten Fremd-DNA identisch ist, hybridisiert werden.
8. Die Einzel-Plaque-gereinigten und als Rekombinanten identifizierten Vektor-Viren werden nochmals auf Vorhandensein und/oder Expression der Fremd-DNA geprüft. Rekombinante Vektor-Viren, die die Fremd-DNA stabil enthalten und/oder exprimieren, stehen für die weitere Anwendung zur Verfügung.

Die Abbildungen stellen folgendes dar:
- Fig. 1: Physikalische Karte des Genoms des EHV-2 Stammes Thein 400/3 für die Restriktionsendonuklease EcoRI. Die Position der repetitiven DNA Sequenzen, die innerhalb der DNA Sequenzen der EcoRI DNA Fragmente B, C, G, I, J, L und M lokalisiert wurden, sind als schwarze Boxen gekennzeichnet. Map unit = Genomkoordinate; kbp = Kilobasenpaare.
- Fig. 2: Darstellung der physikalischen Eigenschaften des Shuttle Vektors pX2-EH2-C1 (6.3 kbp,, der 3 kbp der beiden terminalen Regionen des EcoRI DNA Fragments C des EHV-2 Stammes Thein 400/3 beherbergt.
- Fig. 3: Darstellung der physikalischen Eigenschaften des Shuttle Vektors pEH2-EBt-X2 (4.6 kbp), der 1956 bp des rechten Terminus des EcoRI DNA Fragments B des EHV-2 Stammes Thein 400/3 beherbergt.
- Fig. 4: DNA Nukleotidsequenz der rechten Flanke des EcoRI DNA Fragments B des EHV-2 Stammes Thein 400/3 zwischen den Erkennungsstellen für EcoRI (Genomkoordinate 0.190; Nukleotidposition 1) und BglII (Genomkoordinate 0.189; Nukleotidposition 1596).
- Fig. 5: Darstellung der physikalischen Eigenschaften des rekombinierten Plasmids pX2-EH2-C-LZ, in dem die LacZ Kasette (4440 bp) in die HindIII und BamHI Schnittstellen des Shuttle Vektors pX2-EH2-C1 iseriert worden ist.
- Fig. 6: Darstellung der physikalischen Eigenschaften des rekombinierten Plasmids pEH2-EBt-LZ, in dem die LacZ Kasette (4440 bp) in die HindIII und BamHI Schnittstellen des Shuttle Vektors pEH2-EBt-X2 inseriert worden ist.
- Fig. 7: Mikrophotographie von ED-2 Zellkulturen, die mit rekombinierten Equinen Herpesviren infiziert sind, die das bakterielle β-Galaktosidase Gen exprimieren. Die Behandlung der Zellkulturen mit 5-Bromo-4-chloro-3-indolylbeta-D-galactosid (X-gal, 250 ug/ml) führte zur Ausbildung von blauen Plaques. Die Plaquemorphologie und die Intensität der Expression des β-Galaktosidase Gens sind für die rekombinierten Viren EHV-2-B-Lac-7-231 und EHV-2-C-LacZ-658 in Teil A bzw. B gezeigt.
- Fig. 8: Gen für gB von EHV-1 kloniertin pUC 19 (p19-EHV-1-GpB).
- Fig. 9: Gen für gB von EHV-1 kloniert in puC 19 modifiziert durch Insertion von 2 DNA Adaptoren (p 19-EHV-1-gB-M4).
- Fig. 10: Modifizierter Shuttle Vektor gemäß Beispiel 7.4 pEH2-EB-LZ
- Fig. 11: Modifizierter Shuttle Vektor gemäß Beispiel 7.4 pEH2-EC-LZ
- Fig. 12: Modifizierter Shuttle Vektor mit inseriertem EHV-1 gB-Gen pEH2-EB-LZ + EH1 gB
- Fig. 13: Modifizierter Shuttle Vektor mit inseriertem pEH2-EC-LZ + EHlgB

### Beispiele

### Konstruktion von EHV-2 Virusrekombinanten

1. Präparation der EHV-2 DNA
1.1. Anzüchtung des EHV-2- Stammes Thein 400/3 auf Equine Derm Zellen (ED) (zehn) oder mehr Plastikkulturflaschen, 150 cm, 650 ml, Fa. Costar) bei 37°C für 4 bis 6 Tage. Anzuchtmedium: E-MEM 2,0 g (Eagle Minimal Esential Medium 2,0 g NaHCO₃/l), 10 % Foetales Kälberserum (Fa. Flow) (Virol. Arbeitsmethoden Bd. 1, Gustav-Fischer Verlag, Stuttgart 1974, A. Mayr, O.A, Bachmann, B. Bibrack und G. Wittmann).
1.2. Danach wurde der Überstand der infizierten Zellen bei 5.000 Upm zentrifugiert, um Zellbestandteile zu entfernen.
1.3. Der zellfreie Überstand wurde 1 Stunde bei 25.000 Upm (Rotor Beckmann SW-27) ultrazentrifugiert (Fa. Beckmann).
i.4. Virionenpellet wurde in phosphatgepufferte Kochsalzlösung (PBS) (8 g NaCl, 0,2 g H₂HPO₄, 1,44 g NaH₂PO₄, 0,2 KCl, ad 1 l Aqua dest.) oder TNE (0,05 M Tris, 0,1 M NaCl, 0,001 M EDTA, pH 7,2) resuspendiert und über ein Saccharosekissen (30 % wässrige in TNE gepufferte, oder in PBS gepufferte Saccharose) bei 25.000 Upm für 1 Stunde zentrifugiert.
1.5. Das so gereinigte Virionenpellet wurde in 20 ml TNE resuspendiert und mit 2 ml 10 % Natriumdodecylsulfat (SDS) auf eine Endkonzentration von 1 % SDS eingestellt. Durch diese Behandlung werden die Virionen lysiert.
1.6. Zugabe von 100 µg/ml Proteinase K (Fa. Boehringer) und Inkubation bei 37°C für 1 Stunde (Gross-Bellard et al., 1973, Eur. J. Bioch. 36: S. 32).
1.7. Extraktion der DNA durch zweimalige Phenol- und anschließende Chloroform-Isoamylalkohol-Extraktion nach Marmur (1961, J. Mol. Biol. 3: S. 208).
1.8. Überstand wurde mit 8 M Kaliumacetat-Lösung auf eine Konzentration von 0,8 M Kaliumacetat eingestellt und dann mit 3-fachem Volumen von absolutem Ethanol (<-20°C) versetzt. Die Ethanol-DNA-Extrakt-Lösung wurde über Nacht bei -20°C belassen und daraufhin für 30 min bei 6.000 Upm bei -20°C zentrifugiert.
1.9. Nach Dekantieren des Überstandes wurde das DNA-Pellet in 0,01 oder 0,1xSSC (1 x SSC besteht aus: 0,15 M NaCl, 0,015 M Na-Citrat, pH 7,2) gelöst.
1.10 Bestimmung der Reinheit und der Konzentration der DNA wurde photometrisch durch Absorptionsmessung bei 2600 nm und 280 nm Wellenlänge durchgeführt.
1.11 Aufbewahrung der DNA bei 4°C.

2. Etablierung der EHV-2 Genbank
Eine definierte Genbank des EHV-2 Stammes Thein 400/3, die die gesamten viralen DNA-Sequenzen beherbergt, wurde für die Restriktionsendonukleasen EcoRI, HinDIII und BamHI etabliert.
2.1. Restriktion von 10 µg der EHV-2 DNA mit der Restriktionsendonuklease EcoRI (Fa. Boehringer, 20U/µg) unter den vom Hersteller angegebenen Bedingungen.
2.2. Auftrennung der resultierenden Fragmente in der Agarose (Fa, BRL) Gelelektrophorese ((80 V, 20 h, 4°C) nach Sharp et al. (1973, Biochem. 12: 3055-3063).
2.3. Anfärbung des Gels mit Ethidiumbromid (5 µg/ml) und Sichtbarmachung der DNA-Fragmente unter UV-Licht. Präparation der DNA Fragmente durch Ausschneiden der 18 resultierenden DNA Banden (EHV-2 EcoRI DNA Fragmente A bis R) aus dem Agarosegel.
2.4. Elektroelution der DNA-Fragmente nach Sambrook, Fritsch und Maniatis (1989), Molecular Cloning, Cold Spring Harbor, NY. Aufbewahrung bei -20°C.
2.5. Restriktion von 0,1 picoMol (6 x 10¹⁰ Moleküle) der DNA des Plasmidvektors pACYC184 (Chang and Cohen, 1978, K. Bacteriol. 143:1141) mit der Restriktionsendonuklease EcoRI, Anschließend Behandlung mit alkalischer Phosphatase (Fa, Boehringer) nach Sambrook, Fritsch und Maniatis (1989), Molecular Cloning, Cold Spring Harbvor, NY.
2.6. Ligation der individuellen EHV-2 EcoRI DNA-Fragmente mit der EcoRI und alkalischer Phosphatase behandelten DNA des Plasmidvektors pACYC184 unter Zugabe von 2U T4-DNA-Ligase (Fa. Boerhinger) nach Sambrook, Fritsch und Maniatis (1989), Molecular Cloning, Cold Spring Harbor, NY.
2.7. Transformation von kompetenten E.coli C600 Zellen mit dem Ligationsansatz und Selektion der transformierten Bakterienkolonien auf Tetracyclinresistenz nach Sambrook, Fritsch und Maniatis (1989), Molecular Cloning, Cold Spring Harbor, NY.
2.8. Anzüchtung, Reinigung und Charakterisierung der rekombinierten Plasmide, die die EHV-2 EcoRI DNA Fragmente A bis R beinhalten nach Sambrook, Fritsch und Maniatis (1989), Molecular Cloning, Cold Spring Harbor, NY.

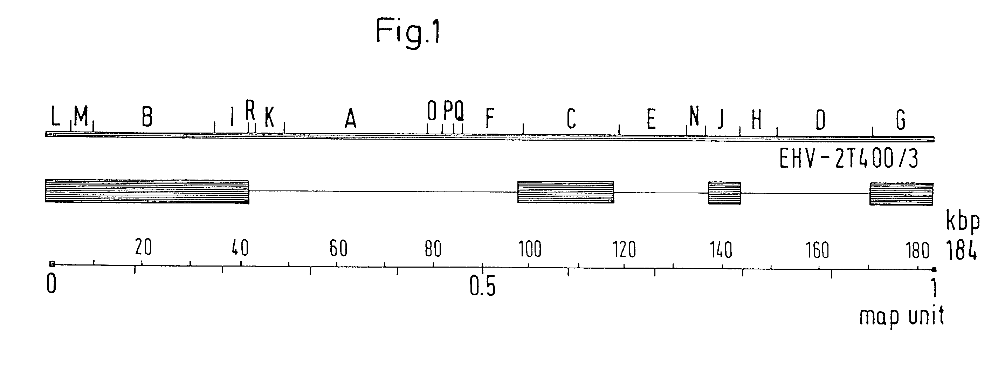

3. Konstruktion der physikalischen Karte des Virusgenoms
Die physikalische Karte des viralen Genoms des EHV-2 Stammes Thein 400/3 (Abb.1) wurde anhand von partieller Behandlung der DNA mit der Restriktionsendonuklease EcoRI sowie Endmarkierungen und DNA/DNA-Hybridisierungen unter Anwendung der viralen Genbank nach Grossmann und Moldave (1989), In: Methods in Virology, ed. S.P. Colowick und N.O. Kaplan, Academic Press, NY, und nach Sambrook, Fritsch und Maniatis (1989), Molecular Cloning, Cold Spring Harbor, NY, konstruiert (Abb. 1). Die Genomkoordinaten der jeweiligen DNA-Fragmente sind in Tabelle 1 aufgeführt.

**Tabelle 1**

| Größe der EcoRI DNA-Fragmente des Genoms des EHV-2 Stammes Thein 400/3 | | |
|---|---|---|
| DNA Fragment | Größe (kbp) | Genomkoordinaten |
| A | 30 | 0.266-0.429 |
| B | 25 | 0.054-0.190 |
| C | 20 | 0.537-0.646 |
| D | 20 | 0.822-0.932 |
| E | 14 | 0.646-0.717 |
| F | 12,5 | 0.469-0.537 |
| G | 12,4* | 0.932-1 |
| H | 8,2 | 0.770-0.822 |
| I | 6,8 | 0.190-0.228 |
| J | 6,8 | 0.741-0.779 |
| K | 6,8 | 0.236-0.266 |
| L | 5,1* | 0. -0.027 |
| M | 5,1 | 0.027-0.054 |
| N | 4,3 | 0.717-0.741 |
| 0 | 2,7 | 0.420-0.445 |
| P | 2,35 | 0.445-0.458 |
| Q | 1,0 | 0.458-0.469 |
| R | 1,4 | 0.228-0.236 |
| Summe: | 183,45 | |

| | | |
|---|---|---|
| * Terminale Fragmente | | |

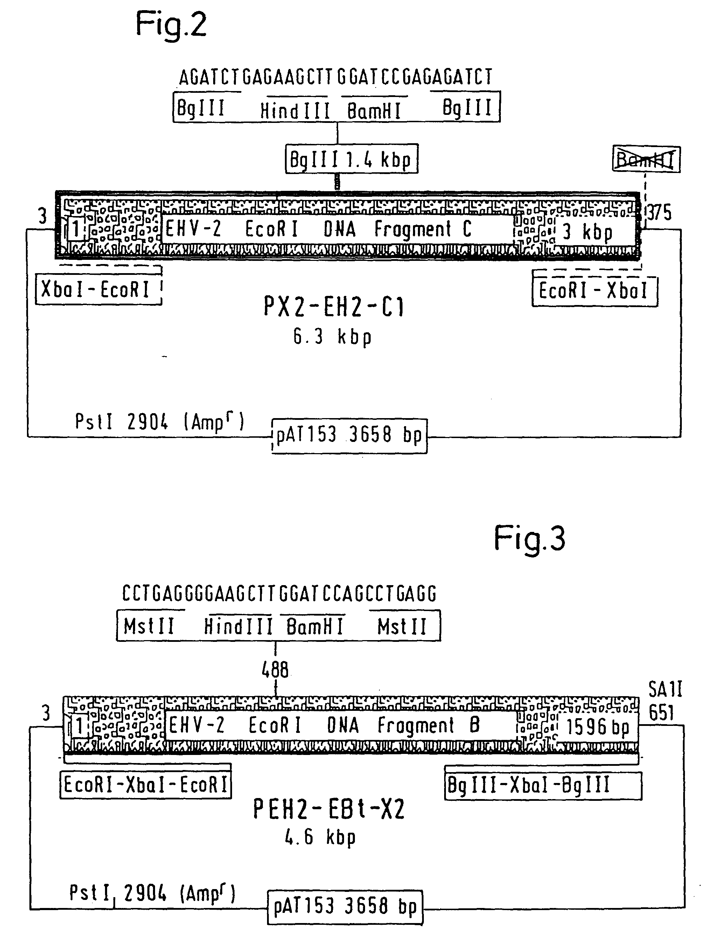

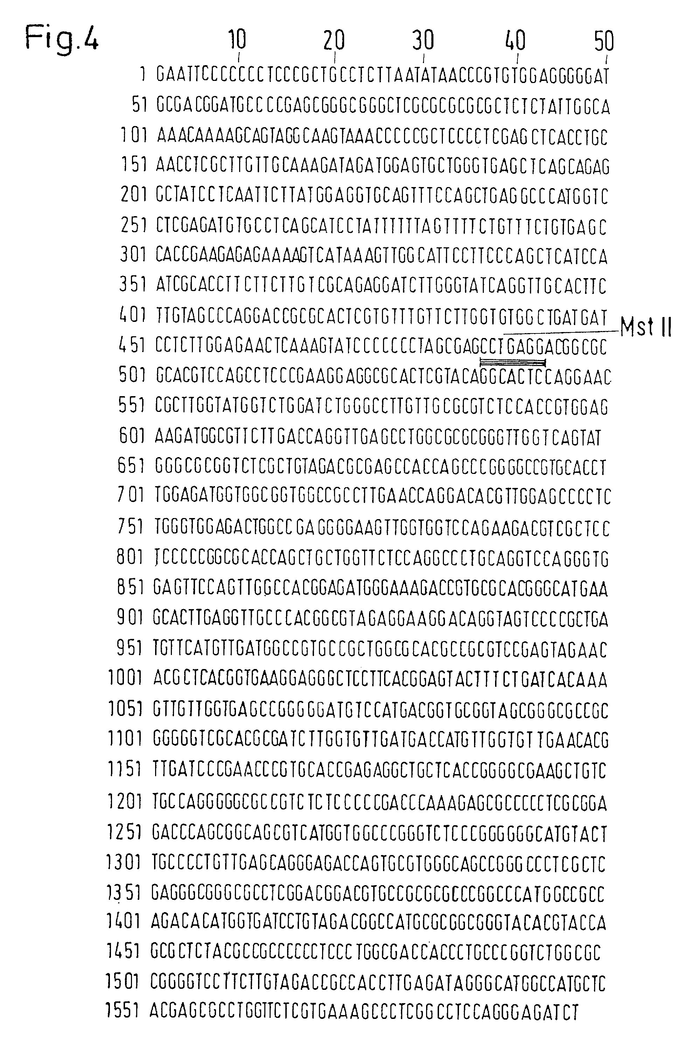

4. Charakterisierung der repetitiven DNA Sequenzen des viralen Genoms
Die repetitiven DNA Sequenzen des viralen Genoms des EHV-2 Stammes Thein 400/3 wurden nach Grossman und Moldave (1980), In: Methods in Virology, ed. S.P. Colowick und N.O. Kaplan, Academic Press,, NY, und Sambrook, Fritsch und Maniatis (1989), Molecular Cioning, Cold Spring Harbor, NY, durch DNAIDNA Hybridisierungen unter Einsatz der viralen Genbank in den EcoRI DNA Fragmenten B, C, G, I, J, L und M lokalisiert. Die Positionen dieser repetitiven DNA Sequenzen sind in Abb. 1 dargestellt.
5. Konstruktion des Shuttle Vektors zur Übertragung fremder genetischer Elemente in das EHV-2 Genom
Zur Inserrierung fremder genetischer Informationen in das EHV-2 Genom wurden zwei Shuttle Vektoren pX2-EH2-C1 (Abb. 2) und pEH2-EBt-X2 (Abb. 3) konstruiert. Beide Shuttle Vektoren haben einen bakteriellen Plasmidanteil von pAT153 (Twigg und Sheratt (1980) Nature 283: 216-19) und einen viralen Anteil von 3 kbp der DNA-Sequenzen des EcoRI DNA Fragments C (pX2-EH2-C1) bzw. 1,6 kbp der DNA Sequenzen des EcoRI DNA Fragments B (pEH2-EBt-X2).
5.1. zu Shuttle Vektor DX2-EH2-C1:
   Das virale Insert des Shuttle Vektors pX2-EH2-C1 beinhaltet die DNA Sequenzen der beiden terminalen Regionen des EcoRI DNA Fragments C, die durch eine Erkennungssequenz der Restriktionsendonuklease BglII begrenzt sind. Die Restriktionsendonuklease BglII trennt die beiden terminalen Regionen in zwei Hälften von 1.4 bzw. 1.6 kbp Größe. An dieser BglII Schnittstelle wurde ein Polylinker (BglII-HindIII-BamHI-BglII) eingefügt, so daß die Inserierung fremder genetischer Elemente ermöglicht wird (siehe Abb. 2). Die Konstruktion des Shuttle Vektors pX2-EH2-C1 erfolgte in folgenden Schritten. Die angewendeten Methoden sind detailliert in Sambrook, Fritsch und Maniatis (1989), Molecular Cloning, Cold Spring Harbor, NY, beschrieben.
   5.1.1. Molekulare Klonierung des EcoRI DNA Fragments C (20 kbp; Genomkoordinaten 0.537-0.646) in den bakteriellen Plasmidvektor pACYC184 und Etablierung des rekombinierten Plasmids pyEH2-E-C.
   5.1.2. Restriktion der DNA des rekombinierten Plasmids pyEH2-E-C mit der Restriktionsendonuklease BglII. Da innerhalb der DNA Sequenzen des pACYC184 keine BglII Schnittstellen vorhanden sind, werden durch diese Behandlung diejenigen DNA Sequenzen innerhalb des EcoRI DNA Fragments C entfernt, die von den äußeren BglII Schnittstellen begrenzt sind. Anschließend Ligation der DNA des deletierten rekombinierten Plasmids.
   5.1.3. Isolierung des Inserts des deletierten rekombinierten Plasmids und molekulare Klonierung in den bakteriellen Plasmidvektor pAT153, in den innerhalb der Erkennungssequenzen für EcoRI und BamHI (pAT153 Nukleotidposition 3 bis 375) ein DNA Linker mit den Schnittstellen für XbaI-EcoRI-XbaI inseriert wurde, wobei die ursprünglichen Schnittstellen für EcoRI und BamHI eliminiert wurden. Etablierung des rekombinierten Plasmids pEH2-E-C.
   5.1.4. Restriktion der DNA des rekombinierten Plasmids pEH2-E-C mit der Restriktionsendonuklease BglII und Inserierung eines DNA Linkers der die Erkennungssequenzen für BglII-HindIII-BamHI-BglII beinhaltet. Etablierung des rekombinierten Plasmids pX2-EH2-C1 (Abb. 2).
5.2 zu Shuttle Vektor DEH2-EBt-X2:
   Das virale Insert des Shuttle Vektors pEH2-EBt-X2 beinhaltet die DNA Sequenzen der rechten Flanke des EcoRI DNA Fragments B (1596 bp), in die an der Nukleotidposition 488 ein Polylinker (MstII-HindIII-BamHI-MstII) eingefügt wurde, der ebenso die Inserierung fremder DNA Elemente erlaubt (siehe Abb. 3).
   Die Konstruktion des Shuttle Vektors pEH2-EBt-X2 erfolgt in folgenden Schritten. Die angewendeten Methoden sind detailliert in Sambrook, Fritsch und Maniatis (1989), Molecular Cloning, Cold Spring Harbor, NY, beschrieben,
   5.2.1. Molekulare Klonierung des rechten Terminus (1596 bp) des EcoRI DNA Fragments B (EcoRI/BglII) in den bakteriellen Plasmidvektor pAT153, in den innerhalb der Erkennungssequenzen für EcoRI und SalI (pAT153 Nukleotidposition 3 bis 650) ein DNA Linker mit den Schnittstellen für XbaI-EcoRI-BglII-XbaI inseriert wurde, wobei die Originalerkennungsstelle für EcoRI in pAT153 eliminiert wurde, Etablierung des rekombinierten Plasmids pEH2-EBt.
   5.2.2. Charakterisierung des rechten Terminus des EcoRI DNA Fragments B durch Bestimmung der DNA Nukleotidsequenz im M13 Phagensystem. Die Nukleotidsequenz der einzelsträngigen DNA der individuellen rekombinierten M13mp18 und 19 Phagen des EcoRI DNA Fragments B wurden nach der Dideoxy-Ketten-Terminationsmethode mit einer modifizierten T7 DNA Polymerase bestimmt (Sanger et al., 1977, Proc, Nat1. Acad. Sci. USA 74: 5463-5467; Tabor und Richardson, 1987, proc. Natl. Acad. Sci. USA 74: 4767-4771). Die DNA Nukleotidsequenz ist in Abb. 4 dargestellt.
   5.2.3. Restriktion der DNA des rekombinierten Plasmids pEH2-E-Bt mit der Restriktionsendonuklease MstI (Nukleotidposition 488 des EHV-2 EcoRI DNA Fragments B). Da innerhalb der DNA Sequenzen des pAT153 keine MstI Schnittstellen vorhanden sind, wird das rekombinierte Plasmid durch diese Behandlung linearisiert. Anschließend Inserierung des DNA Linkers, der die Erkennungssequenzen für MstI-HindIII-BamHI-MstI beinhaltet. Etablierung des rekombinierten Plasmids pEH2-EBt-X2 (Abb. 3).

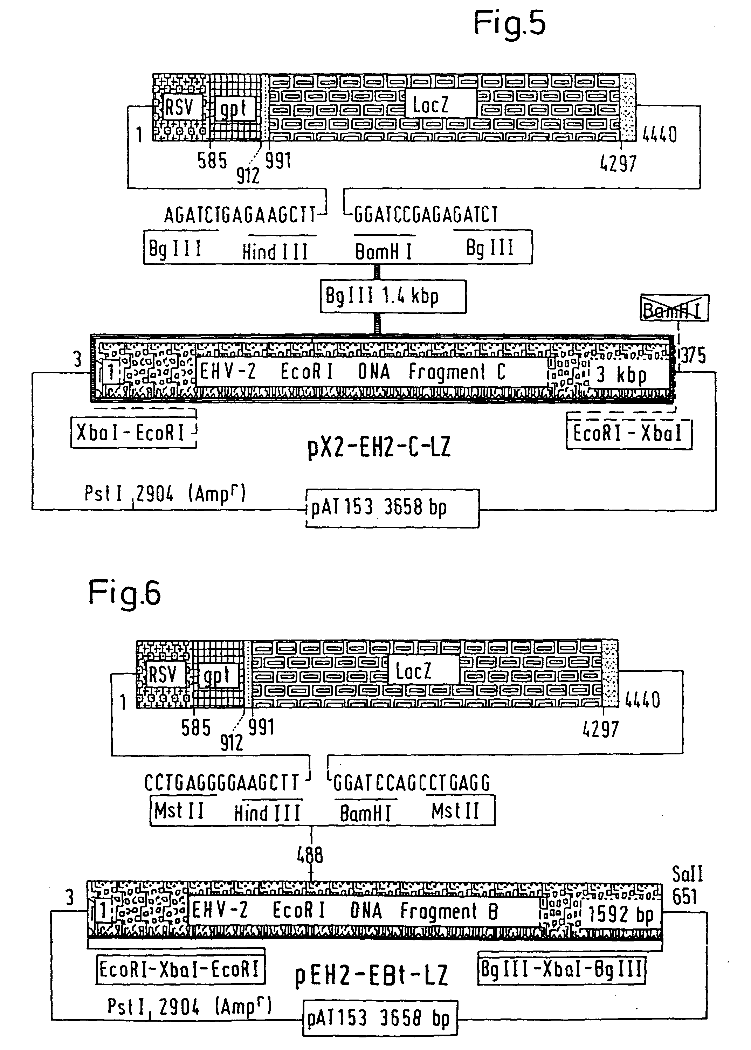

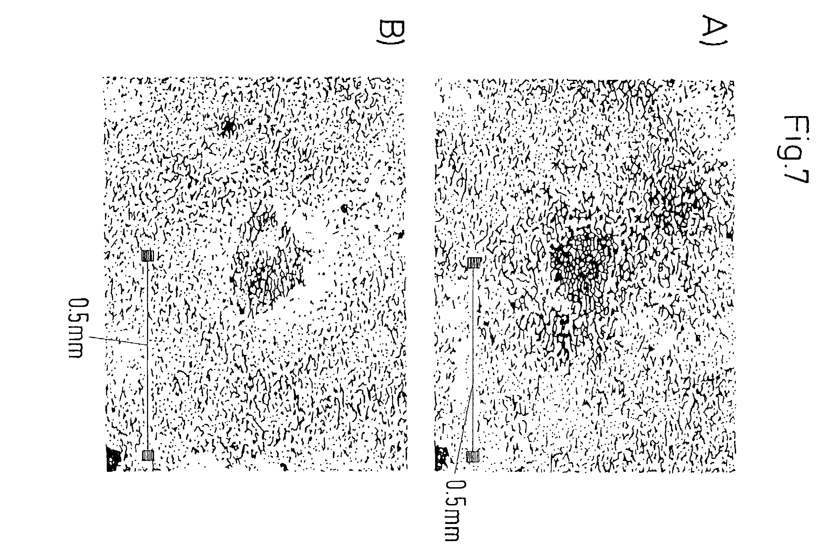

6. Konstruktion eines rekombinierten Equinen Herpesvirus Typ 2, das die bakterielle β-Galaktosidase exprimiert
Das bakterielle β-Galaktosidase Gen (LacZ) wurde als fremde genetische Information ausgewählt, um zu demonstrieren, daß das EHV-2 Genom in der Lage ist, als eukaryontischer Vektor zu fungieren und fremde genetische Elemente zu exprimieren. Die angewendeten Methoden sind detailliert in Sambrook, Fritsch und Maniatis (1989), Molecular Cloning, Cold Spring Harbor, NY, beschrieben. Die Konstruktion erfolgte in folgenden Schritten:
6.1. Das bakterielle β-Galaktosidase Gen (LacZ-Kassette; Hall et al., 1983, J. Mol. Appl. Gen. 2: 101-106; Gorman et al., 1983, Science 221: 551-553) wurde als HindIII/BamHI Fragment aus dem rekombinierten Plasmid pH-BB-LacZ (Rösen-Wolff et al., 1991, Virus Research, im Druck) isoliert.
6.2. Die LacZ-Kassette wurde in die HindIII und BamHI Erkennungssequenz des Shuttle Vektors pC2-EH2-C1 (Abb. 2) inseriert. Das rekombinierte Plasmid pX2-EH2-C-LZ (Abb. 5) resultiert aus der Inserierung der LacZ-Kasette in den Shuttle-Vektor pX2-EH2-C1. Dieses Konstrukt erlaubt die Isolierung des gesamten Inserts des rekombinierten Plasmids mit den EHV-2 Flanken und der LacZ-Kassette durch die Behandlung mit der Restriktionsendonuklease XbaI.
6.3. Die LacZ-Kassette wurde in die HindIII und BamHI Erkennungssequenz des Shuttle Vektors pEH2-EBt-X2 (Abbs. 3) inseriert. Das rekombinierte Plasmid pEH2-EBt-LZ (Abb. 6) resultiert aus der Inserierung der LacZ-Kassette in den Shuttle Vektor pEH2-EBt-X2, Dieses Konstrukt erlaubt die Isolierung des gesamten Inserts des rekombinierten Plasmids mit den EHV-2 Flanken und der LacZ-Kassette durch die Behandlung mit der Restriktionsendonuklease XbaI.
6.4. Die Transferierung der LacZ-Kassette in das EHV-2 Genom wurde durch DNA Kotransfektion nach Sambrook, Fritsch und Maniatis (1989), Molecular Cloning, Cold Spring Harbor, NY, durchgeführt. Für die Transfektion wurden 1 bis 3 picoMol DNA des Inserts des rekombinierten Plasmids pX2-EH2-LZ oder pEH2-Bt-LZ mit 0,01 bis 0,1 picoMol nativer EHV-2 T400/3 DNA nach der Calcium-Phosphat Methode (Graham und van der Eb, 1973, Virology 52: 456-467) kopräzipitiert.
6.5. Die kopräzipitierte DNA wurde zur Transfektion von ED Zellkulturen eingesetzt. Die transfizierten Zellkulturen wurden bei 37°C inkubiert. Die transfizierten Zellen wurden täglich auf das Auftreten von cytopathischen Effekten (CPE) untersucht.
6.6. Beim Auftreten von CPE wurde die Virusrekombinanten selektiert. Die Selektion solcher Virusrekombinanten, die β-Galaktosidase exprimieren, wurde nach dem Blue-Plaque-Verfahren durchgeführt. Die transfizierten Zellkulturen, die nach der Transfektion vereinzelt Virusplaques beherbergen, wurden mit 1 %iger Agarose (Fa. BRL) in PBS, die 250 µg/ml der chromogenen Substanz 5-Bromo-4-chloro-3-indolyl-β-D-galactosid (X-Gal, Fa. Boehringer) beinhaltete, überschichtet. X-Gal führt bei β-Galaktosidase Akitvität zur Blaufärbung der betreffenden Visrusplaques innerhalb von vier bis 8 Stunden.
6.7. Blaue Einzelplaques wurden mit einer sterilen Glaskanüle (Durchmesser 1 mm) angestochen und auf ED Zellen angezüchtet. Dieses Vorgehen wurde dreimal zur Reinigung von rekombinierten Virusstämmen wiederholt. Die DNA Sequenzen des β-Galaktosidase Gens im EHV-2 Genom der Virus-rekombinanten wurde durch DNA/DNA Hybridisierungen nach Sambrook, Fritsch und Maniatis (1989), Molecular Cloning, Cold Spring Harbor, NY, nachgewiesen. Damit gelang es folgende rekombinierte Virusstämme zu etablieren:
   6.7.1. Rekombiniertes Virus EHV-2-C-LacZ-658, das die LacZ-Kassette in seinem Genom innerhalb der DNA Sequenzen des EcoRI DNA Fragments C des EHV-2 Genoms beherbergt und in der Lage ist, dieses fremde Gen zu exprimieren. Die Expression der β-Galaktosidase der individuellen Virusplaques kann wie unter Punkt 6.6. beschrieben durch die Blaufärbung nach der Behandlung mit X-Gal nachgewiesen werden. Ein Beispiel ist in Abb. 7B dargestellt.
   6.7.2. Rekombiniertes Virus EHV-2-B-LacZ-231, das die LacZ-Kassette in seinem Genom innerhalb der DNA Sequenzen des EcoRI DNA Fragments B des EHV-2 Genoms beherbergt und in der Lage ist, dieses fremde Gen zu exprimieren. Die Expression der β-Galaktosidase der individuellen Virusplaque kann wie unter Punkt 6.6, beschrieben durch die Blaufärbung nach der Behandlung mit X-Gal nachgewiesen werden. Ein Beispiel ist in Abb. 7A dargestellt.

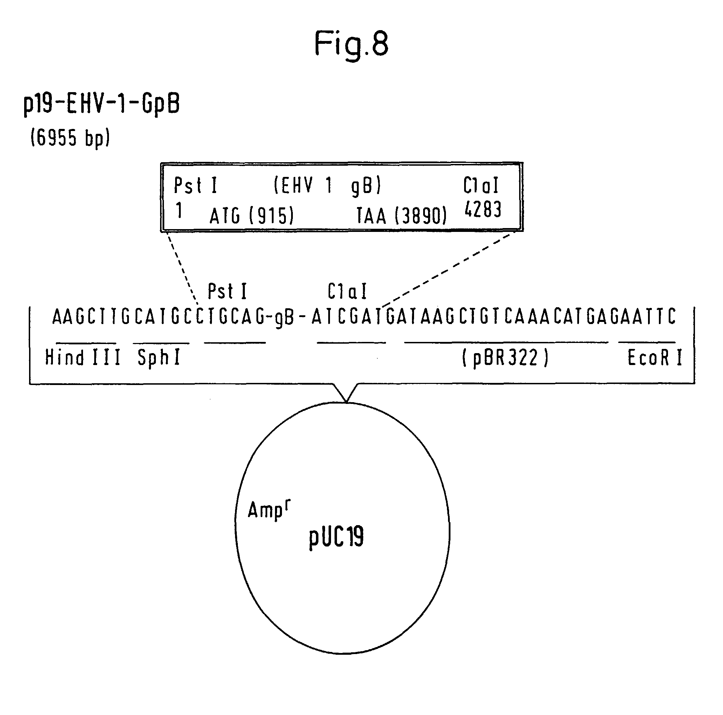

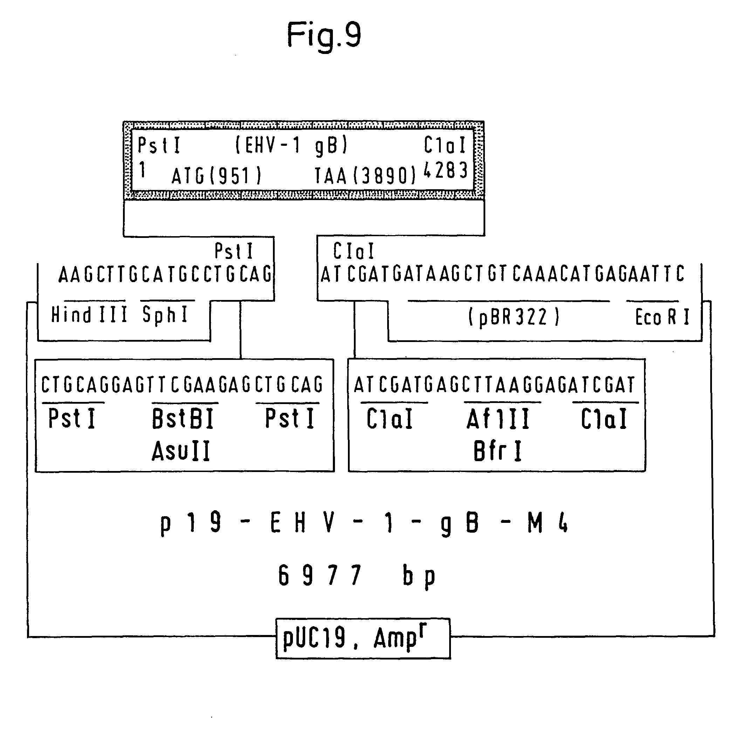

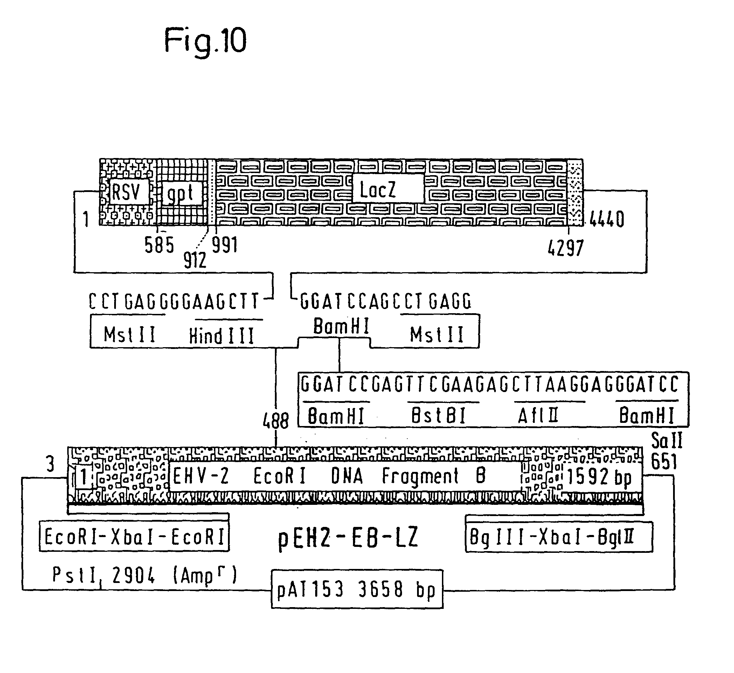

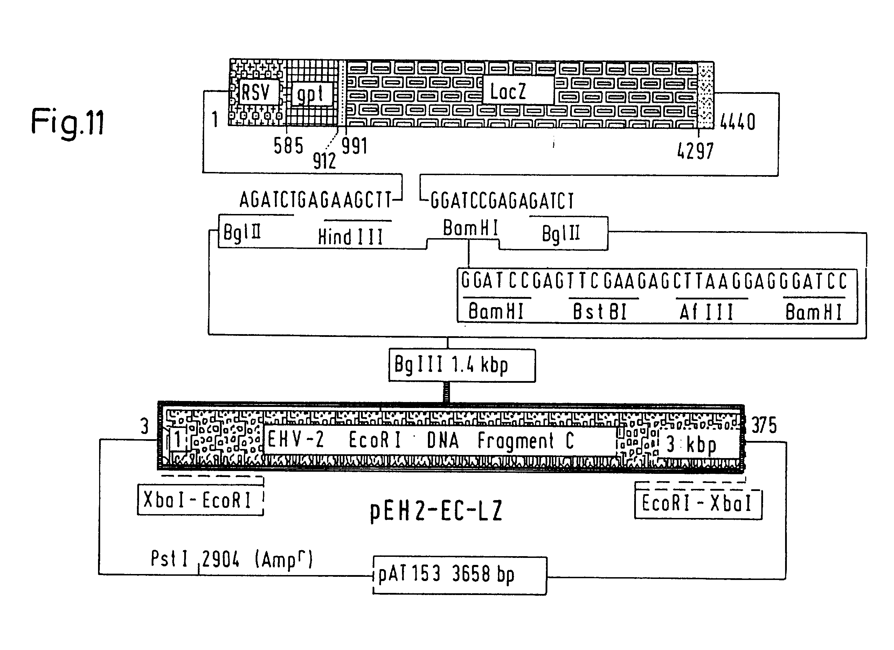

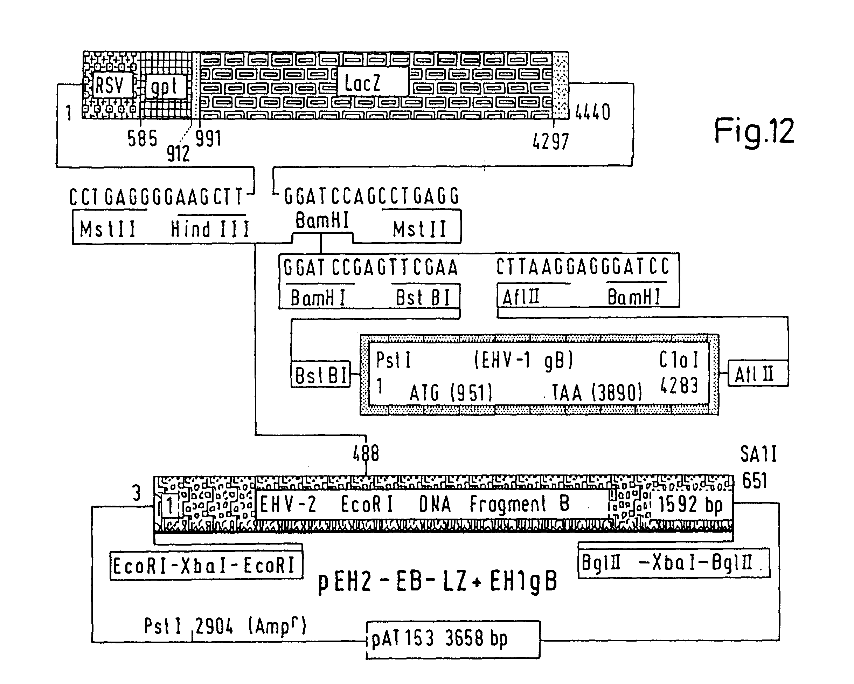

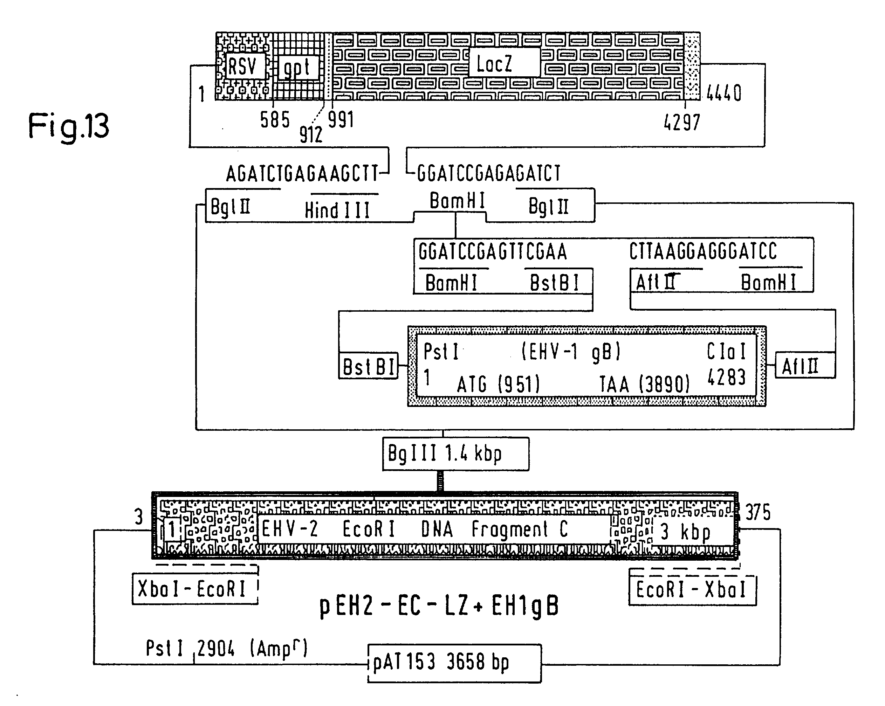

7. Konstruktion von EHV-2 Virusrekombinanten, die das Gen für das Glykoprotein gB von EHV-1 enthalten.
7.1 Vermehrung und Reinigung von EHV-1 Stamm Mar87
   Als Vertreter von EHV-1 wurde der Stamm Mar87 (am 24.7.1990 hinterlegt beim Institut Pasteur C.N.C.M. unter der Nummer I-980) gewählt. Das Virus wurde auf der permanenten Zell-Linie Equine Derm (ED) in der Gewebekultur bei 37°C vermehrt. Als Zellkulturmedium diente E-MEM (Virol.Arbeitsmethoden Bd.1, Gustav Fischer Verlag, Stuttgart 1974, A.Mayr - P.A.Bachmann - B.Bibrack - G.Wittmann), das mit 2,0 g NaHCO₃ pro Liter komplettiert und bei der Zellvermehrung mit 10 % FCS (fötales Kälberserum) versetzt wurde. Das Virus-haltige Medium wurde bei 100% CPE geerntet und bei 5000xg zur Entfernung von Zelltrümmern zentrifugiert. Aus dem Zentrifugationsüberstand wurden die Viruspartikel bei 25.000 UpM im Rotor SW-27 (Fa.Beckmann) für 1 Stunde in der Ultrazentrifuge pelletiert. Das Viruspellet wurde in PBS (8g NACl; 0,2g K₂HPO₄; 1,44g Na₂HPO₄, 0,2g KCl; (Aquadest. ad 1 Liter) oder in 1xTEN (50mM Tris; 0,1M NaCl; 1mM EDTA; pH7,2) resuspendiert und über ein Saccharose-Kissen (30% Saccharose in PBS oder 1xTEN) bei 25.000 UpM im Rotor SW-27 für 1 Stunde durch Ultrazentrifugation erneut pelletiert und in 1xTEN resuspendiert.
7.2 Reinigung der genomischen DNA von EHV-1 und Klonierung der BamHI-DNA-Fragmente A und I
   Die in 1xTEN resuspendierten Viruspartikel wurden durch Zugabe einer wässrigen 10%igen Natriumdodecylsulfatlösung (SDS), auf eine SDS-Endkonzentration von 1% lysiert und virales Protein nach Zugabe von ProteinaseK (Fa.Boehringer, Endkonzentration 100 µg/ml) und Inkubation für 1 Stunde bei 37°C abgebaut (Gross-Bellard et al., Eur.J.Biochem.36, 32ff). Die Extraktion der genomischen DNA erfolgte durch zweimalige Phenol- und anschließende Chloroform-Isoamylalkohol Extraktion (Marmur 1961, J.Mol.Biol.3, 208ff). Der wäßrige Oberstand wurde auf 0,8M Kaliumacetat eingestellt, mit de dreifachen Volumen Äthanol versetzt und über Nacht bei -20°C gela gert. Nach Sedimentation der gefällten DNA durch Zentrifugation bei 10.000xg wurde das DNA-Pellet in 0,01xSSC oder 0,1xSSC (1xSSC 0,15M NaCl; 0,015M Na-Citrat; pH7,2) gelöst.
   Die gereinigte DNA von EHV-1 wurde mit dem Restriktionsenzym BamHI nach Angaben des Herstellers (Boehringer Mannheim) restringiert und die entstandenen DNA-Fragmente im Agarose-Gel elektrophoretisch getrennt (80V; 20Stunden; 4°C) (Sharp et al., 1973, Biochem.12, 3055-3063). Die DNA-Fragmente BamHI-A (22kb) und -I (7kb) wurden durch Elektroelution aus dem Agarose-Gel isoliert (Molecular Cloning; ed. Sambrook, Fritsch und Maniatis; Cold Spring Harbor, N.Y.; 1989) und beide Fragmente jeweils im PlasmidVektor pAT153 (Twigg,A und Sheratt, 1980, Nature 283, 216-218) in die BamHI-Schnittstelle des Vektors molekular kloniert (Molecular Cloning; ed. Sambrook, Fritsch und Maniatis; Cold Spring Harbor, N.Y.; 1989).
7.3 Klonierung des Gens von EHV-1 für das Glykoprotein gB in pUC19
   Das terminale BamHI/PstI-DNA-Fragment mit 1388 bp des BamHI-Fragmentes A von EHV-1 wurde isoliert. Hierzu wurde die DNA des BamHI-A Klons von EHV-1 mit BamHI und PstI restringiert, die Fragmente im Agarose-Gel elektrophoretisch getrennt und das DNA-Fragment mit 1388 bp durch Elektroelution gewonnen (Molecular Cloning; ed. Sambrook, Fritsch und Maniatis; Cold Spring Harbor, N.Y.; 1989). Ebenso wurde das terminale BamHI/ClaI-DNA-Fragment mit 2901 bp des BamHI-Fragmentes I von EHV-1 durch Inkubation der DNA des BamHI-I Klons von EHV-1 mit den Restriktionsenzymen BamHI und ClaI (Double Digest, Molecular Cloning; ed. Sambrook, Fritsch und Maniatis; Cold Spring Harbor, N.Y.; 1989), elektrophoretischer Auftrennung der entstandenen DNA-Fragmente im Agarose-Gel und anschließender Elektroelution des DNA-Fragmentes mit 2901 bp isoliert.
   Die isolierten DNA-Fragmente wurden in den Plasmid-Vektor pUC19 so inseriert, daß das hieraus entstandene rekombinante Plasmid das intakte Gen für gB von EHV-1 enthielt. Hierzu wurde die DNA des Vektors pUC19 mit EcoRI und PstI (Double Digest) geöffnet und ein chemisch synthetisierter DNA-Adapter mit einer terminalen EcoRI-Erkennungssequenz, einer terminalen PstI-Erkennungsequenz und einer internen ClaI-Erkennungssequenz in den geöffneten Vektor an die EcoRI- und PstI-Schnittstelle inseriert (Molecular Cloning; ed. Sambrook, Fritsch und Maniatis; Cold Spring Harbor, N.Y.; 1989). Die DNA des so modifizierten Plasmid-Vektors wurde mit PstI und ClaI wiederum restringiert und anschließend die geöffnete Plasmid-DNA mit dem isolierten BamHI/PstI-DNA-Fragment (1388 bp) des BamHI-Fragmentes A, dem isolierten BamHI/ClaI-DNA-Fragment (2901 bp) des BamHI-Fragmentes I von EHV-1 und dem Enzym T4-Ligase zur Inserierung beider EHV-1 DNA-Fragmente inkubiert (Molecular Cloning; ed. Sambrook, Fritsch und Maniatis; Cold Spring Harbor, N.Y.; 1989). Das so hergestellte rekombinante Plasmid mit 6955 bp, das das intakte Gen für gB von EHV-1 als 4283 bp Insert (ATG an Position 915, TAA an Position 3890) enthält, wurde als p19-EHV-1-GpB bezeichnet (siehe Abbildung 8 ).
   Anschließend wurde in das rekombinante Plasmid p19-EHV-1-GpB ein DNA-Adapter mit der doppelsträngigen Desoxyribonukleotidsequenz mit folgender Basensequenz 
5'-GGAGTTCGAAGAGCTGCA-3'
3'-ACGTCCTCAAGCTTCTCG-5'
 mit flankierenden PstI-Sites und interner BstBI- (AusII-)Site chemisch svnthetisiert und in die PstI-Erkennungssequenz des Plasmids pl9-EHV-1-GpB inseriert. Hierfür wurde das Plasmid mit PstI geschnitten und das linearisierte Plasmid mit dem doppelsträngigen DNA-Adapter und T4-Ligase inkubiert.
   Ein zweiter doppelsträngiger DNA-Adapter mit der Nukleotidsequenz 
5'-CGATGAGCTTAAGGAGAT-3'
   3'-GACTCGAATTCCTCTAGC-5'
 mit flankierenden ClaI-Sites und interner AflII- (BfrI-)Site wurde chemisch synthetisiert und in die ClaI-Site von p19-EHV-1-GpB inseriert. Hierfür wurde das mit dem ersten Adapter versehene Plasmid mit ClaI behandelt und anschließend mit dem zweiten doppelsträngigen Adapter und T4-Ligase inkubiert. Das neu entstandene Plasmid wurde mit p19-EHV-1-gB-M4 bezeichnet (Abbildung 9).
7.4 Präparation von Shuttle-Vektoren zur Inserierung des Gens für gB von EHV-1 in das Genom von EHV-2
   Die doppelsträngige DNA-Sequenz mit der Basensequenz 
5'-GATCCGAGTTCGAAGAGCTTAAGGAGG-3'
— 3'-GCTCAAGCTTCTCGAATTCCTCCCTAG-5'
 mit flankierenden BamHI-Sites und interner BstBI-(AsuII-) und BfrI-(AflII-)Site wurde chemisch synthetisiert und als doppelsträngiger DNA-Adapter in den in Bsp.6.3 beschriebenen Shuttle-Vektor pEH2-EBt-LZ, der das Marker-Gen für lacZ bereits beherbergte, in die BamHI-Erkennungssequenz inseriert.
   Hierfür wurde pEH2-EBt-LZ mit BamHI behandelt und anschließend mit T4-Ligase und dem hier beschriebenen doppelsträngigen DNA-Adapter inkubiert. Es entstand das Plasmid pEH2-EB-LZ (siehε- Abbildung 10).
   Der gleiche doppelsträngige DNA-Adapter mit flankierenden BamHI-Sites und interner BstBI-(AsuII-) und BfrI-(AflII-)Site wurde als doppelsträngiger DNA-Adapter in die BamHI-Site des rekombinierten Plasmids pX2-EH2-C-LZ (s.Bsp.6.2 oben), das das Marker-Gen für lacZ bereits beherbergte, durch Behandlung des pX2-EH2-C-LZ mit BamHI und anschließender Inkubation der geschnittenen Plasmid-DNA mit der synthetisch hergestellten DNA-Sequenz und T4-Ligase inseriert. Es entstand das Plasmid pEH2-EC-LZ (siehe Abbildung 11).
   Das Gen von EHV-1 für gB aus p19-EHV-1-gB-M4 wurde isoliert zur Inserierung in die Shuttle-Vektoren pEH2-EC-LZ und pEH2-EB-LZ. Hierfür wurde das Plasmid p19-EHV-1-gB-M4 mit den Restriktionsenzymen BstBI und BfrI im Double Digest behandelt und die entstandenen DNA-Fragmente im Agarose-Gel elektrophoretisch getrennt (Sharp et al., 1973, Biochem.12, 3055-3063). Das DNA-Fragment mit 4283 bp, das das Gen für gB von EHV-1 enthält, wurde durch Elektroelution aus dem Gel isoliert (Molecular Cloning; ed. Sambrook, Fritsch und Maniatis; Cold Spring Harbor, N.Y.; 1989).
   Das 4283 bp DNA-Fragment mit dem Gen für gB von EHV-1 wurde nun jeweils in die BstBI- und BfrI-Erkennungssequenzen der zwei rekom binanten Plasmide pEH2-EC-LZ und pEH2-EB-LZ inseriert. Die DNA de rekombinanten Plasmids pEH2-EB-LZ wurde im Double Digest mit den Restriktionsenzymen BstBI und BfrI behandelt. Die restringierte und somit linearisierte Plasmid-DNA wurde mit dem 4,28 kbp DNA-Fragment und T4-Ligase inkubiert, das Gen für gB von EHV-1 somit inseriert. Es entstand der Shuttle-Vektor pEH2-EB-LZ-EH1gB (siehe Abbildung 12 zur Übertragung der Gene für gB von EHV-1 und lacZ von E.coli in das Genom von EHV-2.
   In einem zweiten Ansatz wurde die DNA des rekombinanten Plasmids pEH2-EC-LZ im Double Digest mit den Restriktionsenzymen BstBI und BfrI behandelt. Die restringierte und somit linearisierte Plasmid DNA wurde mit dem oben beschriebenen 4,28 kbp DNA-Fragment und T4 Ligase inkubiert, das Gen für gB von EHV-1 somit auch in dieses Plasmid inseriert. Es entstand der zweite Shuttle-Vektor pEH2-EC-LZ+EH1gB (siehe Abbildung 13, ebenfalls zur Übertragung der Gene für gB von EHV-1 und lacZ von E.coli in das Genom von EHV-2.
7.5 Transfektionsversuche mit EHV-2-T400 DNA und Shuttle-Vektor-DNA zur Herstellung von EHV-2 Rekombinanten, die die Gene für gB von EHV-1 und lacZ von E.coli tragen
   Die Transferierung des LacZ Gens und des EHV-1-gB Gens in das EHV-2 Genom wurde durch DNA-Kotransfektion durchgeführt.
   Hierzu wurde zunächst die DNA des Plasmids pEH2-EB-LZ+EH1gB mit XbaI restringiert und die enstandenen Fragmente im Agarose-Gel elektrophoretisch getrennt. Das DNA-Fragment mit 15 kb, das das Insert repräsentierte, wurde elektroeluiert (Sambrook, Fritsch und Maniatis (1989), Molecular Cloning, Cold Spring Harbor, NY).
   Virale DNA von EHV-2 Stamm Thein 400/3 wurde isoliert und im CsCl-Gradienten gereinigt.
   Der Zell-Monolayer konfluenter ED-Zellen, kultiviert in 24-LochPlatten, wurde zweimal mit Medium, einmal mit Medium komplettiert mit DEAE-Dextran (MW 2.000.000, Konzentration 1mg/ml), ein weiteres mal mit Medium komplettiert mit DEAE-Dextran (MW 2.000.000, Konzentration 0,1mg/ml) gewaschen und anschließend mit 100µl Medium komplettiert mit DEAE-Dextran (MW 2.000.000, Konzentration 0,05mg/ml) pro Napf versetzt.
   Auf die Dextran-behandelten ED-Zellen wurden jeweils 1-3 pico Mol DNA des Inserts von Plasmid pEH2-EB-LZ + EHlgB in je 15 µl gegeben pro Napf. Zusätzlich wurden in jedem Napf 0,01 bis 0,1 pico Mol der DNA von EHV-2 Thein 400/3 in je 55 µl zugesetzt.
   In einem zweiten Ansatz wurden die Dextran-behandelten ED-Zellen mit jeweils 1-3 pico Mol DNA des Inserts von Plasmid pEH2-EC-LZ+EH1gB in je25 µl und mit jeweils 0.01 bis 0.1 pico Mol der DNA von EHV-2 Thein 400/3 in je 55 µl versetzt.
   Die Kulturen wurden bei 37°C für 6 Stunden inkubiert.
   Die transfizierten Zellkulturen wurden nun in Medium, das mit 5% FCS komplettiert war, bei 37°C inkubiert und täglich auf das Auftreten von cytopathischen Effekten (CPE) untersucht.
   Beim Auftreten von CPE wurden die Virusrekombinanten selektiert. Die Selektion von Virusrekombinanten, die β-Galaktosidase exprimierten, wurde nach dem Blue-Plaque-Verfahren durchgeführt. Die transfizierten Zellkulturen, die nach der Transfektion vereinzelt Virusplaques bildeten, wurden mit 1%iger Agarose (Fa. BRL) in PBS die 250 µg/ml der chromogenen Substanz 5-Bromo-4-chloro-3-indolyl-β-D-galactosid (X-Gal, Fa. Boehringer) beinhaltete, überschichtet K-Gal führt bei β-Galaktosidase Aktivität zur Blaufärbung der betreffenden Virusplaques innerhalb von vier bis acht Stunden.
   Blaue Einzelplaques wurden mit einer sterilen Glaskanüle (Durchmesser 1 mm) angestochen und auf ED Zellen übertragen. Dieses Vor gehen wurde dreimal zur Reinigung von rekombinierten Virus-stämme wiederholt.
   Die DNA Sequenzen des gB Gens im EHV-2 Genom der Virus-rekombinanten wurden durch DNA/DNA Hybridisierungen nachgewiesen. Hierzu wurden infizierte Zellen auf Nitrozellulose-Filter übertragen, lysiert und fixiert.
   Die EHV-1-gB spezifische Sonde wurde folgendermaßen präpariert: Die DNA des Plasmids p19-EHV-1-gB-M4 wurde mit den Enzymen PstI und ClaI im Double Digest restringiert, die entstandenen DNA-Fragmente im Agarose-Gel elektrophoretisch getrennt und das DNA-Fragment mit 4,28 kbp durch Elektroelution aus dem Gel isoliert. Diese isolierte DNA wurde radioaktiv mit 32p markiert (Methode Rigby et al., 1977, J.Mol.Biol. 114 , 237-256) und als Sonde zum Nachweis von DNA-Sequenzen des Gens für gB von EHV-1 in EHV-2 Rekombinanten in die Hybridisierung eingesetzt.
   So gelang es, rekombinante EHV-2 Stämme zu etablieren, die neben dem Marker-Gen (lacZ) auch das Gen für gB von EHV-1 beherbergen. Zum Beispiel konnten mit Hilfe des Shuttle-Vektors pEH2-EC-LZ+EH1gB die Virus-Rekombinanten EH2LZgB4 und EH2LZgB3, die das gB Gen von EHV-1 enthalten, konstruiert werden.
   So identifizierte EHV-2 Rekombinanten, die über DNA/DNA-Hybridisierungen nachgewiesen DNA-Sequenzen des gB Gens von EHV-1 enthielten, wurden nochmals zur Bildung von Einzel-Plaques auf ED-Zellen inokuliert. So wurden die EHV-2 Rekombinanten EH2LZgB45 und EH2LZgB123 isoliert.
   Die Transkription der Fremd-DNA wurde mittels DNA/RNA-Hybridisierung durchgeführt. Hierzu wurden ED-Zellen mit den Virus-Rekombinanten in einer Infektions-Dosis von 50 Plaque-Bildenden-Einheiten (PFU)/Zelle infiziert. Die RNA aus diesen infizierten Zellen wurde mittels GuanidinHCl und Cäsium-Chlorid nach der Methode von Glisin et al (1974, Biochemistry 106, 492ff) und Ullrich et al (1977, Science 196, 1313ff) extrahiert. Die anschließenden Analysen zur Bestimmung des Gehaltes von EHV-1-gB spezifischer RNA mittels der oben erwähnten EHV-1-gB spezifischen DNA-Sonde wurde nach Lehrach et al (Biochemistry 16, 4743-4751) und "Molecular Cloning", ed. Sambrook, Fritsch und Maniatis; Cold Spring Harbor, N.Y., 1989.
   Zum Nachweis von EHV1-gB spezischer RNA wurde das Insert des Plasmids P19-EHV1gBM4 als Sonde eingesetzt. Es konnte unter Anwendung dieses Verfahrens spezifische RNA-Transkripte in den ED-Zellen, die mit Virus-rekombinanten EH2LZgB45 und EHVLZgB123 infiziert worden waren, nachgewiesen werden. Die Translation der Fremd-DNA wurde immunologisch nachgewiesen.

## Patentansprüche

1. Nicht-virulente oder attenuierte Equine Herpesviren vom Serotyp 2 (EHV-2), die neben den zu ihrer Replikation notwendigen Genomsequenzen mindestens ein fremdes DNA-Element tragen, welches eine Länge von mindestens 20 Nukleotiden aufweist und in einem Fragment inseriert ist, das dem EcoRI-B und/oder -C Fragment des EHV-2 Stammes Thein 400/3, CNCH I-981 entspricht.

2. Equine Herpesviren gemäß Anspruch 1, die mindestens ein fremdes DNA-Element enthalten, das für ein Protein kodiert und/oder durch die Inserierung die Funktion von EHV-Genomsequenzen inaktiviert und/oder das EHV-Genom an einer gewünschten Position markiert.

3. Impfstoffe enthaltend EHV-2 gemäß Anspruch 1.

4. Verwendung von EHV-2 gemäß Anspruch 1 als Vektoren für fremde DNA-Elemente.

5. Verwendung von EHV-2 gemäß Anspruch 1 zur Herstellung von Impfstoffen.

## Claims

1. Non-virulent or attenuated equine herpesviruses of serotype 2 (EHV-2) which, besides the genome sequences necessary for their replication, carry at least one foreign DNA element which has a length of at least 20 nucleotides and is inserted in a fragment which corresponds to the EcoRI-B and/or -C fragment of the EHV-2 strain Thein 400/3, CNCH I-881.

2. Equine herpesviruses according to Claim 1, which contain at least one foreign DNA element which codes for a protein and/or inactivates, owing to the insertion, the function of EHV genome sequences and/or labels the EHV genome at a required position.

3. Vaccines containing EHV-2 according to Claim 1.

4. Use of EHV-2 according to Claim 1, as vectors for foreign DNA elements.

5. Use of EHV-2 according to claim 1 for the preparation of vaccines.

## Revendications

1. Herpès virus équins non virulents ou atténués de type sérologique 2 (EHV-2), qui portent à côté des séquences génomiques nécessaires pour leur réplication, au moins un élément ADN étranger qui présente une longueur d'au moins 20 nucléotides et qui est inséré dans un fragment qui correspond au fragment EcoRI-B et/ou -C de la souche EHV-2 Thein 400/3, CNCHI-981.

2. Herpès virus équins suivant la revendication 1, qui contiennent au moins un élément ADN étranger qui code pour une protéine et/ou inactive par l'insertion, la fonction de séquences génomiques EHV et/ou marque le génome EHV dans une position souhaitée.

3. Vaccins contenant EHV-2 suivant la revendication 1.

4. Utilisation de EHV-2 suivant la revendication 1 comme vecteurs pour des éléments ADN étrangers.

5. Utilisation de EHV-2 suivant la revendication 1 pour la préparation de vaccins.
